Europäisches Patentamt

European Patent Office

Office européen des brevets

(19)

(11) Numéro de publication : **0 452 224 A1**

(12) # DEMANDE DE BREVET EUROPEEN

(21) Numéro de dépôt : 91400982.4

(22) Date de dépôt : 12.04.91

(51) Int. Cl.⁵ : **C12N 15/74, C12N 1/20, C12N 1/21, // (C12N1/20, C12R1:46)**

(30) Priorité : 13.04.90 FR 9004860

(43) Date de publication de la demande :
16.10.91 Bulletin 91/42

(84) Etats contractants désignés :
AT BE CH DE DK ES FR GB GR IT LI LU NL SE

(71) Demandeur : SANOFI
40, Avenue George V
F-75008 Paris (FR)
Demandeur : SOCIETE NATIONALE ELF
AQUITAINE (PRODUCTION)
Tour Elf
F-92078 Paris la Défense Cédex 45 (FR)

(72) Inventeur : Prevost, Fabien
68, rue Bonnat, Bt.D
F-31400 Toulouse (FR)
Inventeur : Ritzenthaler, Paul
Chemin Gayssot
F-31320 Castanet-Tolosan (FR)

(74) Mandataire : Gillard, Marie-Louise et al
Cabinet Beau de Loménie 55, Rue
d'Amsterdam
F-75008 Paris (FR)

(54) **Molécule d'ADN et plasmide comprenant au moins un mécanisme de résistance aux phages, bactéries les contenant et leur utilisation.**

(57) Le plasmide selon l'invention est susceptible de s'hybrider avec une molécule d'ADN comprenant au moins un mécanisme de résistance aux phages et est contenu dans les transconjugants issus du croisement d'une souche donneuse choisie parmi les souches :
— souches Lactococcus lactis ssp cremoris déposées à la C.N.C.M. sous les n° I-939 et I-943,
— souche Lactococcus lactis ssp lactis déposée à la C.N.C.M. sous le n° I-940,
— souches Lactococcus lactis ssp lactis var. diacetylous déposées à la C.N.C.M. sous les n° I-941 et I-942,
et d'une souche réceptrice.
Application : Obtention de bactéries lactiques plus résistantes aux phages.

EP 0 452 224 A1

La présente invention concerne une molécule d'ADN et des plasmides susceptibles de s'hybrider avec celle-ci, porteurs d'au moins un mécanisme de résistance aux phages, les bactéries lactiques contenant cette molécule ou ces plasmides, en particulier les lactocoques appartenant à l'espèce Lactococcus lactis, l'utilisation de certaines souches de ces lactocoques pour transférer, notamment par conjugaison, un mécanisme de résistance aux phages à des souches d'intérêt industriel, en particulier dans l'industrie laitière, et à l'emploi de certaines souches Lactococcus lactis pour obtenir ces plasmides.

Les bactéries lactiques sont impliquées dans l'élaboration et la conservation d'un grand nombre de produits alimentaires, tels que les fromages, le beurre, les yaourts, le saucisson ou la choucroute. Parmi ceux-ci les produits laitiers occupent une place particulièrement importante. La transformation industrielle du lait est faite dans des cuves de fermentation de plus en plus grandes, dans lesquelles l'apparition de phages des bactéries lactiques peut avoir des conséquences graves, voire catastrophiques : variation des caractéristiques, notamment organoleptiques, du produit final ; perte du produit présent dans la cuve et nécessité de décontaminer cette dernière ainsi que les installations environnantes. Il existe donc dans l'industrie laitière un besoin impérieux de nouveaux moyens et de nouvelles méthodes permettant de rendre les bactéries lactiques plus résistantes aux phages.

L'invention concerne une nouvelle molécule d'ADN comprenant au moins un mécanisme de résistance aux phages, caractérisée en ce qu'elle comporte une partie fonctionnelle du fragment HindIII-HindIII d'environ 3,3 kb du plasmide pPF144-1 présent dans la souche Eschérichia coli déposée le 09 Avril 1991 à la C.N.C.M. sous le n°I-1070.

Ce fragment HindIII-HindIII d'environ 3,3 kb, a été isolé à partir du plasmide pPF144 contenu dans la souche Lactococcus lactis ssp lactis, déposé à la C.N.C.M. sour le n° I-945, laquelle est un transconjugant issu du croisement de la souche donneuse Lactococcus lactis ssp lactis S91 déposée à la C.N.C.M. le 12 Avril 1991 sous le n° I-940 et de la souche réceptrice Lactococcus lactis ssp lactis S45, dérivée de la souche Lactococcus lactis ssp lactis C2-LL. Mc. Kay et al, 1977, J. Bacteriol. 257-265. Le fragment est porteur d'un ou plusieurs mécanismes de résistance aux phages. Une partie fonctionnelle de ce fragment désigne ici une partie de celui-ci qui comporte au moins un mécanisme de résistance aux phages.

Cette molécule d'ADN est un fragment d'ADN linéaire ou un plasmide.

L'invention concerne donc un nouveau plasmide comprenant au moins un mécanisme de résistance aux phages, caractérisé en ce qu'il est susceptible de s'hybrider avec la molécule d'ADN définie précédemment et est contenu dans les transconjugants issus du croisement d'une souche donneuse choisie parmi les souches ci-après :

– souches Lactococcus lactis ssp cremoris déposées à la CNCM le 12 avril 1990 sous les n° I-939 et I-943 ;
– la souche Lactococcus lactis ssp lactis déposée à la CNCM le 12 avril 1990 sous le n° I-940 ;
– les souches Lactococcus lactis ssp lactis var. diacetylous déposées à la CNCM le 12 avril 1990 sous les n° I-941 et I-942 et d'une couche réceptrice.

Ces souches donneuses sont résistantes aux phages (Rap+), capables de métaboliser le lactose (Lac+) et sensibles à l'érythromycine (Erys) ainsi qu'à la streptomycine (StrS). On sait que chez les lactocoques la capacité de métaboliser le lactose est en général portée par un plasmide non conjugatif, lequel peut être transféré dans une autre souche sous la forme d'un cointégrat avec un autre plasmide conjugatif. Il est donc commode pour la sélection des transconjugants que la souche réceptrice ne soit pas capable de métaboliser le lactose (Lac⁻) et porte un gène de résistance à au moins un de ces antibiotiques. Une souche réceptrice pratique est par exemple la souche Lactococcus lactis ssp lactis S45, Lac⁻, EryR (plasmidique) et StrR (chromosomique), laquelle permet de faire la sélection des transconjugants par la capacité de métaboliser le lactose et la résistance à l'érythromycine, la résistance à la streptomycine servant à différencier les donneuses et les réceptrices.

Le ou les mécanismes de résistance aux phages portés par ce plasmide confèrent aux souches contenant celui-ci une résistance partielle ou totale à une température de 30°C contre les phages du groupe I d'homologie et les phages du groupe III d'homologie, phages virulents représentatifs de tous ceux susceptibles d'être rencontrés dans les accidents de fermentation (RELANO P. et al (1987) J. Gen. Microbiol., 133, 3053-3063).

A une température de 40°C certains de ceux-là continuent de conférer une résistance partielle ou totale contre ces phages.

Ce plasmide, introduit par conjugaison dans la souche réceptrice, porte un ou plusieurs gènes lui conférant un caractère conjugatif. Il est donc susceptible d'être transféré par conjugaison, mécanisme naturel, dans d'autres souches, par exemple des souches d'intérêt industriel.

Ce plasmide porte éventuellement un ou plusieurs gènes conférant à la bactérie lactique le contenant la capacité de métaboliser le lactose.

Ce plasmide peut également porter un ou plusieurs gènes conférant à la bactérie lactique le contenant une activité bactéricide (vis-à-vis de laquelle elle est immune). Ce caractère est un avantage pour une culture pure ou une culture mixte de bactéries qui contiennent toutes ce plasmide, car il évite la prolifération de bactéries contaminantes.

Un plasmide particulièrement prisé est celui qui a les caractéristiques d'un plasmide choisi parmi :

– le plasmide pPF66 contenu dans la souche Lactococcus lactis ssp lactis déposée à la CNCM le 12 avril 1990 sous le n° I-948 ;

– le plasmide pPF72 contenu dans la souche Lactococcus lactis ssp lactis déposée à la CNCM le 12 avril 1990 sous le n° I-946 ;

– le plasmide pPF107 contenu dans souche Lactococcus lactis ssp lactis déposée à la CNCM le 12 avril 1990 sous le n I-947 ;

– le plasmide pPF118 contenu dans la souche Lactococcus lactis ssp lactis déposée à la CNCM le 12 avril 1990 sous le n° I-944 ;

– le plasmide pPF144 contenu dans la souche Lactococcus lactis ssp lactis déposée à la CNCM le 12 avril 1990 sous le n° I-945 ;

ou dont la séquence présente un degré d'homologie élevé avec celle de l'un des plasmides ci-dessus.

Chacun des 5 plasmides mentionnés ci-dessus, respectivement de 66, 72, 107, 118 et 144kb, a été isolé, par digestion enzymatique de l'ADN génomique et analyse de ce dernier par électrophorèse en champ pulsé sur gel d'agarose, dans un clone de transconjugant obtenu par croisement d'une souche donneuse choisie parmi les souches déjà citées, déposées à la CNCM de l'Institut Pasteur sous respectivement les n° : I-943, I-941, I-942, I-939 et I-940, et de la souche réceptrice Lactococcus lactis ssp lactis S45.

Ces plasmides, dont certains sont manifestement des produits de recombinaison génétique, par exemple des cointégrats, des plasmides de la souche donneuse, ont été caractérisés par leur profil de restriction et éventuellement leur carte de restriction. Chacun de ces plasmides est porteur d'au moins un mécanisme de résistance aux phages, conférant à la bactérie lactique qui le porte une résistance partielle ou totale à 30°C contre les phages du groupe I d'homologie et les phages du groupe III d'homologie, cette résistance étant en général conservée à 40°C. Certains de ces plasmides possèdent de plus des éléments génétiques conférant aux bactéries lactiques qui les contiennent la capacité de métaboliser le lactose ou une activité bactéricide.

Certains des autres plasmides qu'il sera possible d'isoler dans d'autres clones de transconjugants obtenus à partir des mêmes souches donneuses auront une séquence présentant un degré d'homologie élevé avec, et/ou comprenant une partie importante de la séquence des 5 plasmides ci-dessus. D'autres de ces plasmides n'auront ni homologie ni sous-séquence commune avec les 5 plasmides ci-dessus mais présenteront également un mécanisme de résistance aux phages, codé par d'autres séquences que celles de ces derniers.

L'invention a également trait à une bactérie lactique résistante aux phages, de préférence appartenant à l'espèce Lactococcus lactis, qui contient au moins une molécule d'ADN ou un plasmide tel que défini ci-dessus.

Cette molécule d'ADN ou ce plasmide peuvent avoir été introduits dans la bactérie lactique par conjugaison, transformation, fusion de protoplastes ou par une autre méthode de transfert de gènes.

Une bactérie de ce type particulièrement appréciée est celle qui est choisie parmi les souches Lactococcus lactis ssp lactis déposées à la CNCM sous les n° I-944, I-945, I-946, I-947 et I-948.

L'invention concerne aussi l'utilisation d'une souche choisie parmi les 5 souches mentionnées au paragraphe précédent, les souches Lactococcus lactis ssp cremoris déposées à la CNCM sous les n° I-939 et I-943, la souche Lactococcus lactis ssp lactis déposée à la CNCM sous le n° I-940 et les souches Lactococcus lactis ssp lactis var. diacetylous déposées à la CNCM sous les n° I-941 et I-942 pour transmettre par conjugaison, transformation, transduction, fusion de protoplastes ou une autre méthode de transfert de gènes, un mécanisme de résistance aux phages à une souche d'intérêt industriel. Ce mécanisme peut être porté par un plasmide ou par une autre partie du génome de la bactérie. Lorsque celui-là est porté par un plasmide il est avantageux de le transférer par conjugaison.

L'invention a également trait aux souches d'intérêt industriel résistantes aux phages ainsi obtenues.

L'invention concerne aussi l'utilisation d'une souche choisie parmi les souches Lactococcus lactis ssp cremoris déposées à la CNCM sous les n° I-939 et I-943, la souche Lactococcus lactis ssp lactis déposée à la CNCM sous le n° I-940 ainsi que les souches Lactococcus lactis ssp lactis var. diacetylous déposées à la CNCM sous les n° I-941 et I-942, pour obtenir le plasmide défini ci-dessus.

L'invention sera mieux comprise à l'aide des exemples ci-après, qui comprennent des résultats expérimentaux et une discussion de ceux-ci. Certains de ces exemples concernent des expériences effectuées dans le but de réaliser l'invention, d'autres des exemples de réalisation de l'invention donnés bien sûr à titre purement illustratif.

Une grande partie de l'ensemble des techniques décrites dans ces exemples, bien connues de l'homme de l'art, est exposée en détail dans l'ouvrage de Sambrook Fritsch et Maniatis : "Molecular cloning ; a Laboratory manual" publié en 1989 par les éditions Cold Spring Harbor Press à New York (2ème édition).

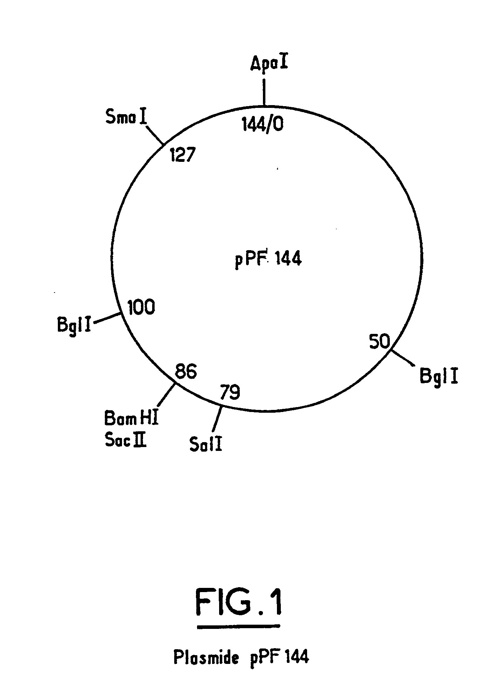

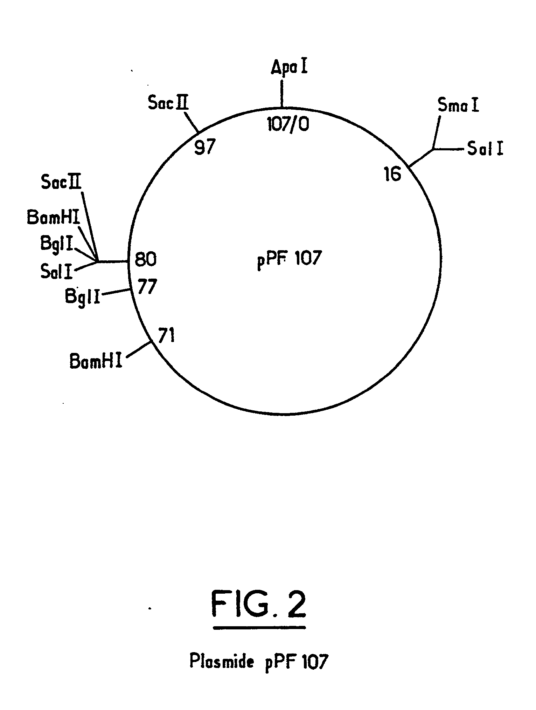

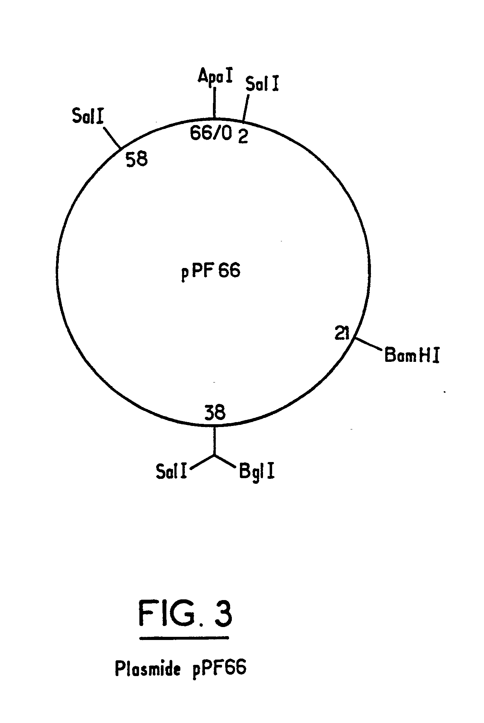

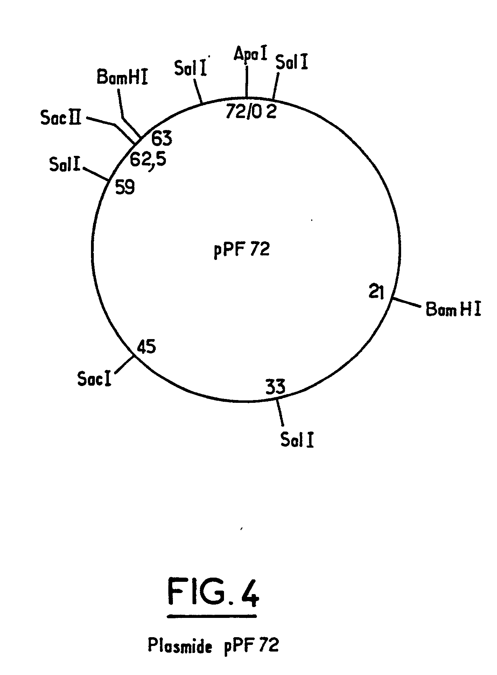

La description ci-après sera mieux comprise à l'aide des figures 1 à 6.

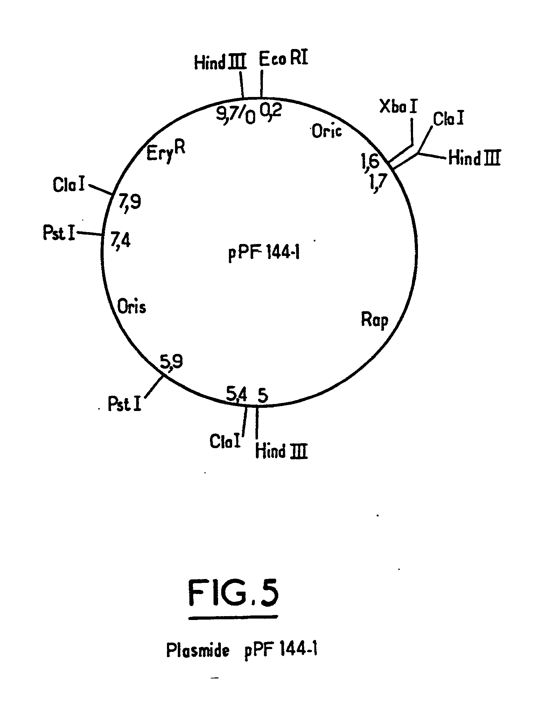

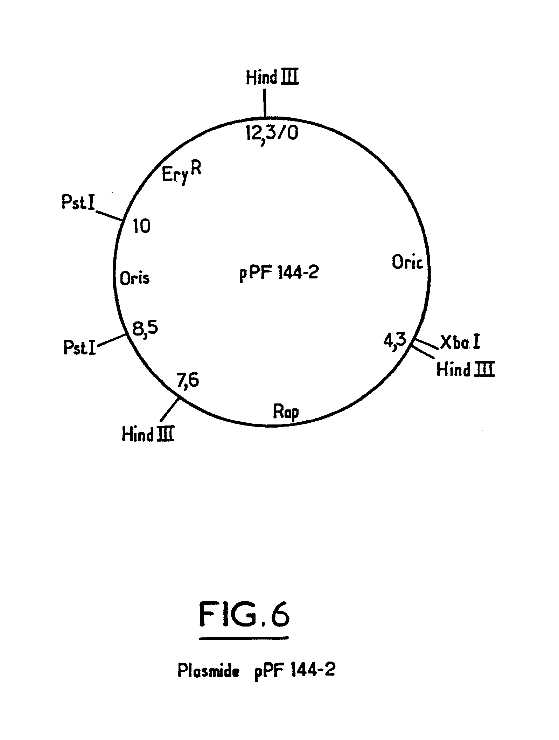

Les figures 1 à 6 représentent respectivement les cartes de restriction des plasmides pPF144, pPF107, pPF66, pPF72, pPF144-1 et pPF144-2, les chiffres vis-à-vis des sites de restriction symbolisant le nombre de kb à partir d'un site particulier pris arbitrairement comme origine. Sur ces figures sont représentés au même endroit les sites de restriction que la sensibilité de la méthode d'analyse n'a pas permis de distinguer. Les figures 5 et 6 indiquent de surcroît de façon approximative les régions où se trouvent l'origine de réplication chez E. coli, notée oric, l'origine de réplication chez les lactocoques, notée oris, le gène de résistance à l'érythromycine, noté Ery$^R$, ainsi que le ou les mécanismes de résistance aux phages, noté Rap.

Exemple 1 :

Criblage de souches de lactocoques très résistantes à un large spectre de bactériophages.

1) Constitution d'une collection de bactériophages et caractérisation partielle de ceux-ci.

On a constitué une collection de bactériophages isolés à partir de lactosérums d'accidents de fermentation, comprenant les phages suivants :
- phage Stl1 décrit par PARADA J.L. et al dans Appl. Environ. Microbiol., 1984, 47, 1352-1354 ;
- phage Ø1358 décrit par JARVIS A.W. et al dans Appl. Environ. Microbiol., 1984, 47, 1031-1038 ;
- phage Ø936 décrit par JARVIS A.W. et al dans Appl. Environ. Microbiolo, 1984, 47, 343-349 ;
- phage P008 décrit par ENGEL G. et al dans Kieler Milchwirtschafliche Forschungsberichte, 1975, 27, 25-48 ;
- phages Ø27, Ø29, Ø30, Ø31, Ø44, Ø49, Ø54, Ø55, Ø56, Ø57, Ø58, Ø59, Ø64, Ø65, Ø70 décrits par RELANO P. et al dans J. Gen. Microbiolo, 1987, 133, 3053-3063 ;
- phages issus de collections privées appelés ici Ø53, Ø60 et Ø40.

Ces phages ont fait l'objet d'une caractérisation au niveau moléculaire : spectre d'hôte, morphologie étudiée au microscope électronique, composition en protéines, profils de restriction de l'ADN et hybridation ADN-ADN.

Ces phages appartiennent aux 3 grands groupes d'homologie (I), (II) et (III), sur la base d'études par hybridation ADN/ADN (RELANO P. et al (1987), J. Gen. Microbiol. 133, 3053-3063). Les groupes (I) et (III) comprennent uniquement des phages virulents. Le groupe (II) comprend des phages virulents et des phages tempérés. A l'intérieur d'un même groupe les homologies sont fortes et, d'un groupe à l'autre, les homologies sont très faibles. Les phages du groupe (I) ont une nucléocapside oblongue alors que les phages des groupes (II) et (III) ont une nucléocapside isométrique.

Cette collection de 22 phages est hautement représentative de l'ensemble des bactériophages que l'on peut rencontrer lors des accidents de fermentations dans les industries laitières.

2) Obtention d'un nouveau stock de phages à partir d'un stock de phages plus ancien.

Chacun des phages de la collection a été multiplié sur sa souche indicatrice (souche sensible au phage considéré) de la façon suivante :

1 ml du milieu de culture de la bactérie indicatrice de densité optique à 600 nm (ci-après DO$_{600}$) d'environ 0,6 (phase exponentielle) est prélevé et mis en présence de 100 µl d'un stock de phages et de chlorure de calcium à 10 mM. L'adsorption du phage sur la bactérie est effectuée pendant 15 min à 30°C selon la bactérie considérée. Puis le tout est dilué quatre fois par du milieu M17 (TERZAGHI et al (1975), Appl. Environ Microbiol. 29, 807-813) contenant du chlorure de calcium à 10 mM, de telle sorte que la DO$_{600}$ finale n'excède pas 0,15. L'ensemble est placé à l'étuve à 30°C jusqu'à ce que la lyse bactérienne soit obtenue. Ce nouveau stock de phages est ensuite centrifugé et le surnageant placé à +4°C en présence de quelques gouttes de chloroforme afin d'éviter le développement de bactéries. Cette méthode donne de bons résultats : on obtient en général entre 10$^9$ et 5.10$^{10}$ phages/ml. Si la lyse n'a pas été obtenue après 6 h d'incubation, 100 µl du surnageant ci-dessus sont prélevés et la manipulation est recommencée à partir de l'adsorption du phage sur la bactérie.

3) Lysotypie.

Souches de lactocoques utilisés :
21 souches Lactococcus lactis ssp cremoris
14 souches Lactococcus lactis ssp lactis
10 souches Lactococcus lactis ssp lactis var. diacetylous
issues de collections privées.
- Protocole de lysotypie :

4

Des cultures de nuit de souches de lactocoques en milieu M17 ont servi à ensemencer des tubes de milieu M17 de telle sorte qu'il y ait une phase exponentielle de croissance ($DO_{600}$=0,5) 300 µl de chaque culture sont mélangés avec 3 ml d'agar mou M17 et du chlorure de calcium à 10 mM.

L'ensemble est étalé sur une boîte de milieu M17 gélosé contenant du chlorure de calcium à 10 mM. 5 µl de stock de phages dans du milieu de culture sont déposés sur ces tapis. Ces boîtes sont déposées en étuve à 30°C pendant une nuit. Dans tous les cas où une lyse est observée, 5 µl d'une dilution au 1/100ème sont également testés. Lorsque aucune lyse n'est observée à cette dilution, deux tests sont effectués :

– 5 µl de stock de phages sont mélangés directement avec 300 µl de culture bactérienne en phase exponentielle de croissance et du chlorure de calcium à 10 mM et étalés sur une boîte de milieu gélosé contenant du chlorure de calcium. Les boîtes sont ensuite placées à l'étuve pendant une nuit : on observe l'apparition éventuelle de plages de lyse.

– le surnageant de culture de la bactérie ayant servi à faire le stock de phages est stérilisé par un passage à travers un filtre de 0,45 µm. Il est ensuite testé sur un tapis de bactéries comme un stock de phages. L'apparition ou non d'un halo est ensuite déterminée.

Résultats : sur l'ensemble des 45 souches de lactocoques testées, 12 souches sont complètement résistantes à tous les phages testés, (7 L. lactis ssp cremoris, 2 L. lactis ssp lactis, 3 L. lactis ssp lactis var diacetylous), 9 souches sont relativement sensibles aux phages mais ne permettent pas le développement de ceux-ci, (les 5 µl de stock de phages inhibent le développement des bactéries lorsque ceux-ci sont déposés sous forme d'une goutte mais si ces derniers sont mélangés au tapis bactérien aucune plage de lyse n'est détectée) (6 L. lactis ssp cremoris, 1 L. lactis ssp lactis, 2 L. lactis ssp lactis var. diacetylous) et 24 souches sont sensibles à au moins un phage.

Exemple 2 :

Transfert par conjugaison d'éléments génétiques impliqués dans la résistance aux phages - Obtention de clones de 5 transconjugants résistants aux phages.

A) Principales techniques expérimentales utilisées :

1) conjugaison (premier cycle de conjugaison) :

- souche réceptrice : Lactococcus lactis ssp lactis S45, Lac⁻, $Ery^R$, $Str^R$ contenant 1) le plasmide pVA838 (MACRINA F.L. et al (1982), Gène, 19, 345-353) qui porte la résistance à l'érythromycine $Ery^R$, et 2) un marqueur chromosomique : la résistance à la Streptomycine $Str^R$.

La souche S45 a été obtenue à partir de la souche S124 (elle-même obtenue à partir de la souche L. lactis ssp lactis C2 comme exposé à l'exemple 3) de la façon suivante.

La souche S124 a été traitée aux ultraviolets selon le protocole décrit par L.L. Mc. KAY (L.L. Mc. KAY et al (1973) Appl. Microbiol. 25, 682-684) de façon à être curée des deux prophages décrits par J.D. EFSTATHIOU et LL. Mc. KAY (J.D. EFSTATHIOU et LL. Mc. KAY (1977) Journal of Bacteriology, 257-265). En a ensuite étalé la souche sur des boîtes de milieu M17 (TERZAGHI B.E. et al (1975) Appl. Env. Microbiol. 29, 807-813) gélosé contenant 500 µg/ml de streptomycine et 0,5% de glucose. Un clone résistant à la streptomycine a été transformé par le plasmide pVA 838 selon une technique proche de celle décrite par POWELL I.B. et al (POWELL I.B. et al (1988) Appl. Environ. Microbiol., 54, 655). Le protocole précis utilisé est exposé ci-après. La souche obtenue est la souche S45.

Protocole de transformation des lactocoques par électroporation

10 ml de milieu M17 à 20 mM de DL thréonine sont ensemencés avec 200 µl d'une culture de nuit. L'incubation est réalisée à 30°C jusqu'à une densité optique à 600 nm d'une valeur de 0,4. Puis cette culture est centrifugée. Le culot est repris par 10 ml de tampon SMP (saccharose 240 mM, $MgCl_2$ 1 mM, tampon phosphate 7 mM pH 6,5). L'ensemble est centrifugé. Le culot est resuspendu dans 900 µl de tampon SMP. 800 µl de cellules sont prélevées auxquelles sont ajoutés 5 µl d'une quantité connue d'ADN : l'ensemble est disposé dans une cuve à électrode et laissé 2 min à +4°C. Le choc électrique est réalisé avec 25 µF et 2,5 kV ("Gene pulser", Bio Rad). Les cellules sont laissées 10 min à +4°C, puis 0,8 ml de M17 x 2 contenant du glucose à 0,4% est ajouté et le tout est disposé dans un tube Eppendorf. L'incubation est effectuée pendant 1 h à +30°C puis les cellules sont centrifugées. Le culot est resuspendu dans le liquide résiduel, mélangé à de la gélose molle contenant du milieu de culture, du glucose à 0,4% et l'antibiotique approprié et l'ensemble est étalé sur une boîte de milieu gélosé contenant également glucose et antibiotique. L'incubation est effectuée pendant 24 h à 30°C.

- souches donneuses :
  - les 12 souches de l'exemple 1 complétement résistantes à tous les phages testés
  - 8 des 12 souches de l'exemple 1 sensibles aux phages mais ne permettant pas le développement de ceux-ci (une souche de ce type a été éliminée car elle n'avait pas une croissance suffisante).

Ces 20 souches portent le caractère de résistance aux phages Rap et la capacité de fermenter le lactose Lac. Elles sont Rap$^+$, Lac$^+$, Ery$^s$ et Str$^s$.

5 parmi ces 20 couches, les souches <u>Lactococcus lactis ssp cremoris</u> S77 et S114, la souche <u>Lactococcus lactis ssp lactis</u> S91 ainsi que les souches <u>Lactococcus lactis ssp lactis var. diacetylous</u> S94 et S96 ont été retenues (v. B) ci-après pour leur capacité de donner des transconjugants portant les caractères Rap et Lac. Elles ont été déposées auprès de la CNCM (Collection Nationale de Culture de Microorganismes de l'Institut Pasteur) respectivement sous les n° I-939, I-943, I-940, I-941 et I-942. Certaines caractéristiques de ces souches sont exposées ci-après dans le tableau I ci-après.

EP 0 452 224 A1

## Tableau 1

Caractéristiques biochimiques des souches S77, S91, S94, S96 et S114 testées avec
la galerie API* 50 CH [API S.A.] et le milieu API 50 CHL [API S.A.]

| Souches \ hydrate de carbone | 4 | 5 | 6 | 10 | 11 | 12 | 13 | 18 | 22 | 24 | 25 | 26 | 27 | 28 | 29 | 32 | 36 | 39 |
|---|---|---|---|---|---|---|---|---|---|---|---|---|---|---|---|---|---|---|
| S77 | | [+] | | + | + | + | + | [+] | + | | | | + | [+] | + | + | | |
| S91 | [+] | + | + | + | + | + | + | + | + | | + | [+] | + | + | + | + | [+] | |
| S94 | | + | | + | + | + | + | | + | [+] | + | + | + | + | + | + | [+] | |
| S96 | | + | | + | + | + | + | | + | [+] | + | + | + | + | + | + | + | + |
| S114 | | | | + | + | + | | | + | | | | | | + | | | |

4 : L Arabinose 5 : Ribose 6 : SXylose 10 : Galactose 11 : Glucose 12 : Fructose 13 : Mannose

18 : Mannitol 22 : N acétylglucosamine 24 : Arbutine 25 : Esculine 26 : Salicine 27 : Cellobiose

28 : Maltose 29 : Lactose 32 : Trebalose 36 : Amidon 39 : Gentiobiose

+ : Utilisation de l'hydrate de carbone par le micro-organisme après 24 h d'incubation en anaérobiose

[+] : Utilisation partielle de l'hydrate de carbone par le micro-organisme après 24 h d'incubation en aérobiose

Les hydrates de carbone de la galerie API 50 CH ne correspondant pas aux numéros cités ci-dessus ne sont pas métabolisés

* moyen bien connu de l'homme de métier pour caractériser les souches par leurs propriétés biochimiques.

La souche S91 possède une bonne cinétique de croissance à 30 et 40°C en milieu M17, mais a une légère inhibition de croissance en présence de 4% NaCl à 30°C en milieu M17.

\* les souches S94 et S96 possèdent une bonne cinétique de croissance à 30°C en milieu M17. A 40°C, cette croissance est très faible, de même qu'en présence de 4% NaCl à 30°C en milieu M17.

\* Les souches S77 et S114 ont une faible cinétique de croissance à 30°C en milieu M17. On n'observe pas de croissance à 40°C, ni même en présence de 4% de NaCl à 30°C en milieu M17.

La sélection des transconjugants se fait par la capacité de fermenter le lactose et la résistance à l'érythromycine, la résistance à la streptomycine servant à différencier les donneuses et les réceptrices.

Protocole de conjugaison :

A partir de culture de nuit en milieu M17, avec une quantité de glucose variant selon la souche considérée, des souches réceptrice et donneuse, 66 µl de la souche réceptrice et 133 µl de la souche donneuse sont mélangés. Ces bactéries sont ensuite étalées sur des boîtes de Pétri contenant du lait écrémé à 11%, du glucose à 0,4% et de l'agar. L'incubation est effectuée pendant 16 h à 30°C, afin que la conjugaison s'effectue. 1 ml de lait écrémé reconstitué à 11% contenant 4% de lactose est étalé puis le lait contenant les bactéries est prélevé. Des fractions de 200 µl ou des dilutions sont enfin étalées sur des boîtes de milieu Elli 1, milieu Elliker modifié (de composition et préparation : bactotryptone 20 g, extrait de levure 5 g, protéose peptone 2,5 g, D[+] lactose 10 g, NaCl 4 g, acétate Na 1,5 g, acide ascorbique 0,5 g, agar 15 g, $H_2O$ q.s.p. 1 l, pH 6,8, stérilisation 15 min à +120°C - cf. ELLIKER P.R. et al (1956), J. Dairy Sci., 39, 1611-1612), additionné d'Erythromycine à 5 µg/ml afin de sélectionner les souches réceptrices et du pourpre de bromocrésol (KAY Mc. et al, (1972) Appl. Microbiol. 23, 1090-1096) afin de repérer parmi les réceptrices les transconjugants Lac$^+$ de couleur jaune. Ces expériences permettent notamment de déterminer l'efficacité théorique de conjugaison définie comme le rapport entre le nombre de transconjugants obtenus et le nombre de cellules réceptrices ajoutées au début de l'expérience de conjugaison ainsi que d'estimer l'efficacité réelle de conjugaison égale à l'efficacité théorique de conjugaison multipliée par l'inverse du facteur de multiplication des réceptrices pendant la conjugaison.

2) Recherche de transconjugants résistants aux phages.

Chaque transconjugant issu du croisement par conjugaison entre une souche donneuse et une souche réceptrice est réisolé sur boîte de milieu Elli-1 additionné de 5 µg/ml d'Erythromycine et de pourpre de bromocrésol, afin d'obtenir une culture pure. Puis 300 µl de cette dernière en phase exponentielle de croissance ($DO_{600}$=0,5) sont étalés, en présence de 3 ml de milieu M17 gélosé contenant du chlorure de calcium 10 mM, sur une boîte de milieu M17 contenant du chlorure de calcium 10mM. 5 µl de stock de phages ou 5 µl de différentes dilutions de ce stock de phages, sont déposés sur ce tapis de cellules. La même expérience est réalisée avec la souche réceptrice témoin S45, la seule différence étant qu'on rajoute du glucose à 0,4% dans le milieu de culture. Ces boîtes sont déposées à l'étuve à 30°C pendant une nuit. Un mécanisme de résistance à un phage particulier dans le transconjugant est visualisé par la différence du titre de phages entre le transconjugant et la souche S45 pour ce phage, et également par la différence de la taille des plages de lyse pour ce phage. Le titre de phages T, également appelé titre phagique, qui représente le nombre de phages actifs sur la souche testée par ml de stock de phages déposé sur le tapis de cellules, exprimé en PFU/ml (PFU = Plaque Forming Unit) est calculé à l'aide de la formule $T = N \times 2 \times 10^{x+2}$ dans laquelle N représente le nombre de plages de lyse obtenues à la dilution $10^{-x}$, x étant le paramètre de dilution. Le diamètre des plages de lyse est par ailleurs mesuré. Ce paramètre donne des indications quantitatives sur le rendement unitaire, nombre de phages obtenus après un cycle de multiplication phagique pour un phage qui infecte une bactérie. Plus la plage de lyse est grande plus le rendement unitaire est élevé.

Les phages utilisés pour mettre en évidence les mécanismes de résistance aux phages sont les phages 053 et 024 (groupe I d'homologie ainsi que le phage 059 (groupe III d'homologie).

B) Résultats.

1) Transfert par conjugaison de la capacité de fermenter le lactose.

On a observé comme Mc Kay et al (Mc KAY et al (1980) Appl. Env. Microbiol. 40, 84-91) que toutes les souches ne sont pas capables de transférer la capacité de fermenter le lactose. Sur les 20 croisements effectués en premier cycle de conjugaison, seuls 6 croisements ont donné des transconjugants, 2 avec les souches L. lactis ssp lactis S88 et S91), 2 avec les souches L. lactis ssp cremoris S77 et S114, ainsi que 2 avec les souches L. lactis ssp lactis var. diacetylous S94 et S96. Le tableau 2 ci-après précise l'efficacité théorique Et de transfert de la capacité de fermenter le lactose, définie comme le rapport entre le nombre de transconjugants obtenus et le nombre initial de réceptrices, pour les 6 souches ci-dessus. Le témoin est constitué par la souche S45 seule.

Tableau 2

Efficacité théorique du transfert par conjugaison de la capacité de fermenter le lactose.

| Souche donneuse | Et |
|-----------------|-----|
| S77 | $5,5 \times 10^{-7}$ |
| S91 | $4 \times 10^{-8}$ |
| S96 | $1,5 \times 10^{-5}$ |
| S88 | $4 \times 10^{-8}$ |
| S94 | $1,2 \times 10^{-7}$ |
| S114 | $4 \times 10^{-8}$ |
| S45 | $< 8 \times 10^{-9}$ |

La souche donneuse la plus efficace pour le transfert par conjugaison de la capacité de fermenter le lactose est la souche S96.

D'autre part, on a vérifié par extraction du contenu plasmidique des transconjugants et analyse par électrophorèse en champ continu (cf. exemple 3) la présence dans chacun de ceux-ci du plasmide pVA838.

Un seul des 6 transconjugants, celui issu de croisement S88 x S45, s'est révélé sensible à l'action de la streptomycine : il semble qu'il y ait eu transfert du plasmide pVA838, en principe non conjugatif, de la souche S45 vers la souche S88. Ce transconjugant a été éliminé.

On a retenu pour les analyses ultérieures les 5 transconjugants issus des croisement S77 x S45, S91 x S45, S96 x S45, S94 x S45 et S114 x S45, appelés par la suite S45-77, S45-91, S45-96, S45-94 et S45-114.

2) Recherche de transconjugants résistants aux phages :

Les principaux résultats obtenus avec les transconjugants retenus, en mettant en oeuvre la technique décrite en A)2), sont rassemblés dans le tableau 3 ci-après, lequel précise pour ces derniers et la souche S45 (témoin) le titre phagique et le diamètre de la plage de lyse.

## Tableau 3

Résistance aux phages des transconjugants Lac$^+$ à une température de 30°C.

| Souche | Phage | | |
| --- | --- | --- | --- |
| | titre phagique (nombre de phages/ml)/diamètre de la plage de lyse (mm) | | |
| | 024 (groupe I) | 053 (groupe I) | 059 (groupe III) |
| S45-91 clone 1 | 0/0 | $4 \times 10^7$/<0,25 | 0/0 |
| S45-96 clone 1 | 0/0 | 0/0 | 0/0 |
| S45-77 clone 6 | $8 \times 10^7$/<0,25 | $2 \times 10^{10}$/<0,25 | 0/0 |
| S45-114 clone 1 | $8 \times 10^6$/<0,25 | $2 \times 10^9$/<0,25 | 0/0 |
| S45-94 clone 1 | 0/0 | $10^{10}$/<0,25 | 0/0 |
| S45(témoin) | $2 \times 10^9$/2 | $2 \times 10^{10}$/3 | $3 \times 10^9$/2 |

On observe à la lecture du tableau ci-dessus :

– Le transconjugant S45-96 clone 1 résiste aux 3 phages testés ;

– les transconjugants S45-91 clone 1 et S45-94 clone 1 sont résistants vis-à-vis des phages 024 et 059 et partiellement résistants vis-à-vis du phage 053 (titre phagique inférieur à celui de la souche témoin et plage de lyse de diamètre inférieur à celle-ci) ;

– les transconjugants S45-77 clone 6 et S45-114 clone 1 sont résistants vis-à-vis du phage 059 et partiellement résistants vis-à-vis des phages 024 et 053.

Remarquons par ailleurs que, pour le transconjugant S45-77, 13 clones ont été testés parmi lesquels seuls 2 (dont le clone 6) présentaient un mécanisme de résistance aux phages. Ce dernier n'est donc pas forcément transféré en même temps que la capacité de fermenter le lactose. Celle-ci, probablement présente sur un plasmide, n'est pas nécessairement sur le même plasmide que le mécanisme de résistance aux phages, lequel étant conjugatif mobilise lors de la conjugaison le plasmide portant la capacité de fermenter le lactose par formation d'un cointégrat.

Les transconjugants S45-91 clone 1, S45-96 clone 1, S45-77 clone 6, S45-114 clone 1 et S45-94 clone 1 ont été déposés auprès de la CNCM (Collection Nationale de Culture de Microorganismes de l'Institut Pasteur) respectivement sous les n° I-945, I-947, I-944, I-948 et I-946.

Exemple 3 :

Mise en évidence des plasmides portant des mécanismes de résistance aux phages.

A) Principales techniques expérimentales utilisées.

1) Curage des souches de leurs plasmides.

Une gamme de concentrations en novobiocine dans du milieu M17 contenant du glucose à 0,4% est constituée. Chacun de ces tubes est ensemencé de telle sorte qu'il y ait $10^5$ cellules/ml. L'incubation est effectuée pendant 18 h à +30°C. Puis le contenu des 2 tubes dont la concentration est la plus proche de la concentration minimale inhibitrice (CMI), est étalé à l'anse de platine sur du milieu M17 contenant du glucose à 0,4%. Après incubation, les clones sont testés pour différents caractères comme exposé ci-après :

2) Test des caractères après traitement à la novobiocine sur plaque de microtitration.

Dans des plaques de microtitration (96 cuves de 500 µl, COSTAR) 100 µl de milieu M17 contenant du glucose et un clone prélevé à l'anse de platine sont placés dans chaque cupule. L'incubation est effectuée à l'étuve de telle sorte que l'on ait une phase exponentielle de croissance.

Puis, par réplique, 2 à 3 µl de chaque culture sont déposés sur des boîtes de Pétri contenant différents milieux-tests :
  – milieu Elli-1 + pourpre de bromocrésol (0,004%) : test de la capacité à fermenter le lactose ;
  – milieu M17 = 5 µg/ml érythromycine + glucose (0,4%) : test de la résistance à l'érythromycine ;
  – milieu M17 + 500 µg/ml streptomycine + glucose (0,4%) : test de la résistance à la streptomycine ;
  – milieu M17 + $CaCl_2$ + $10^7$ phages 059 (groupe III) étalés à l'anse de platine + glucose (0,4%) : test de la résistance aux phages.

Ce phage a été choisi car tous les mécanismes de résistance aux phages sont totalement actifs vis-à-vis de ce phage (cf. exemple 2).

3) Extraction des plasmides des souches.

Les plasmides sont extraits par une technique de lyse alcaline. Une culture de nuit des couches est effectuée dans 5 ml de milieu de culture M17 en présence ou non d'antibiotiques et/ou de glucose à 0,4% (poids/volume). Le même volume de milieu M17 x 2 est ajouté et les cultures sont laissées à l'étuve pendant 1 h. Elles sont ensuite centrifugées. Le culot est resuspendu dans le liquide résiduel. Le tout est recentrifugé en centrifugeuse à tubes Eppendorf. Le surnageant est jeté et le culot est resuspendu à la pointe de pipette avec 900 µl de TES (Tris 100 mM, EDTA 20 mM, saccharose 20%) contenant du lysozyme à 4 mg/ml ; une incubation est effectuée 30 min à 37°C puis les cellules sont centrifugées. Le culot est repris par 300 µl de SDS 1%. NaOH 0,15 N et mélangé à la pointe de pipette puis laissé 10 min à +25°C. Puis sont ajoutés 225 µl d'acétate de sodium 3M, pH 4,8 : 10 min à +4°C. L'ensemble est centrifugé à 10 000 g pendant 10 min à +4°C. Le surnageant est traité volume à volume avec un mélange phénol/$CHCl_3$/alcool isoamylique (24/23/1) puis centrifugé à 10 000 g 1 min à +25°C. La phase aqueuse est précipitée avec 2,5 volumes d'éthanol pendant 30 min à -70°C. Le mélange est centrifugé à 10 000 g 10 min à +4°C, le culot est lavé avec de l'éthanol à 70% puis lyophilisé et repris par 50 µl d'eau. 5 µl de plasmides sont déposés sur gel d'agarose. Cet ADN est suffisamment pur pour être traité par des enzymes de restriction.

4) Deuxième cycle de conjugaison :

- souche réceptrice : Lactococcus lactis ssp lactis S124, Lac⁻, $Ery^R$, Str^s contenant le plasmide pVA838.

La souche S124 a été obtenue à partir de la souche Lactococcus ssp lactis C2 (LL. Mc. KAY et al (1977) J. Bacteriol. 257-265) de la façon suivante.

La souche L. lactis ssp lactis C2 a été traitée à la N-méthyl-N'-nitro-N-nitrosoguanidine, comme cela est décrit par Mc. Kay et ses collaborateurs (1970). (LL. Mc. KAY et al J. Bacteriol. 102, 804-809). Les cellules traitées ont été étalées sur le milieu Elli-1 + pourpre de bromocrésol + lactose 0,5% pour sélectionner les Lac⁻. Une souche Lac⁻, ayant perdu les 5 plasmides que possédait la souche C2, a été transformée avec le plasmide pVA838 selon le protocole de transformation exposé à l'exemple 2. La nouvelle souche obtenue est appelée S124.

- Souches donneuses : - certaines souches transconjugantes du premier cycle de conjugaison portant un mécanisme de résistance aux phages , curées du plasmide pVA838.

La sélection des transconjugants se fait par la capacité de fermenter le lactose et la résistance à l'érythromycine, la résistance à la streptomycine servant à différencier les donneuses et les réceptrices.

Le protocole de conjugaison est celui utilisé à l'exemple 1.

EP 0 452 224 A1

B) Résultats.

1) Curage plasmidique des transconjugants :

L'élimination des plasmides par la novobiocine doit apporter au moins deux informations importantes :
– si un caractère disparaît après curage, un gène ou plusieurs des gènes responsables de ce caractère sont probablement portés par un ou des plasmides ;
– la ségrégation de la capacité à fermenter le lactose par rapport à la résistance aux phages, indépendante ou dépendante, peut nous indiquer que le ou les gènes responsables de ces deux phénotypes ne sont pas ou sont, respectivement portés par le même plasmide.

Après traitement à la novobiocine pour chaque souche testée, on a examiné, dans au moins 100 clones de chacun des transconjugants retenus précédemment, trois caractères :
* capacité à fermenter le lactose,
* résistance à l'érythromycine portée par le plasmide pVA838,
* résistance au phage 059 (groupe III d'homologie).

La résistance à l'érythromycine sert de témoin interne de Curage.

Les résultats obtenus pour les transconjugants retenus, la souche S45 (témoin) et les souches donneuses, traités à la novobiocine (cf. b) ci-après sont rassemblés dans le tableau 4 suivant :

## Tableau 4

Pourcentage du nombre de clones possédant après traitement à la novobiocine un phénotype particulier par rapport au nombre total de clones testés.

| Souche | Phénotype | | | | | |
|---|---|---|---|---|---|---|
| | $Rap^+ Lac^+$ | $Rap^- Lac^-$ | $Rap^+ Lac^-$ | $Rap^- Lac^+$ | $Ery^R$ | $Ery^S$ |
| S45-91 clone 1 | 48 | 52 | 0 | 0 | 72 | 28 |
| S45-96 clone 1 | 100 | 0 | 0 | 0 | 43 | 57 |

**12**

## Tableau 4 (suite)

| Souche | Phénotype | | | | | |
|---|---|---|---|---|---|---|
| | $Rap^+ Lac^+$ | $Rap^- Lac^-$ | $Rap^+ Lac^-$ | $Rap^- Lac^+$ | $Ery^R$ | $Ery^S$ |
| S45-77 clone 6 | 96 | 4 | 0 | 0 | 78 | 22 |
| S45-114 clone 1 | 0 | 44 | 12 | 44 | 74 | 26 |
| S45-94 clone 1 | 11 | 2 | 87 | 0 | 37 | 63 |
| S45 | 0 | 100 | 0 | 0 | 39 | 61 |
| S91 | 38 | 0 | 62 | 0 | / | / |
| S96 | 46 | 0 | 54 | 0 | / | / |
| S77 | 100 | 0 | 0 | 0 | / | / |
| S114 | 100 | 0 | 0 | 0 | / | / |
| S94 | 42 | 0 | 58 | 0 | / | / |

Rap : résistance aux phages ; Lac : capacité de fermenter le lactose ;

$Ery^R$ : résistance à l'érythromycine ;

$Ery^S$ : sensibilité à l'érythromycine.

On constate à la lecture du tableau 4 :

– les caractères Rap et Lac sont présents dans tous les clones du transconjugant S45-96 clone 1. On ne peut toutefois en déduire que les gènes responsables de ces deux phénotypes ne sont pas portés par des plasmides : il arrive en effet que certains plasmides soient résistants au traitement à la novobiocine. L'efficacité de la novobiocine ne peut être mise en cause par le curage du plasmide pVA838 s'est effectué dans de bonnes proportions (57%).

– Les transconjugants S45-91 clone 1, S45-77 clone 6, S45-114 clone 1 et S45-94 clone 1 ont effectivement perdu après curage, dans une certaine proportion, les deux caractères. Ces résultats indiquent l'origine plasmidique probable des caractères Lac et Rap chez ces 4 transconjugants.

– Les transconjugants S45-91 clone 1, S45-96 clone 1 et S45-77 clone 1 ne donnent après curage aucune cellule Rap$^+$ Lac$^-$ ou Rap$^-$ Lac$^+$. Cette ségrégation des deux caractères laisse supposer que les gènes responsables des phénotypes Lac et Rap sont portés par un seul et même plasmide.

– Les transconjugants S45-94 et S45-114 donnent après curage des souches Rap$^+$ Lac$^-$ et/ou Rap$^-$ Lac$^+$. Cette ségrégation indique que les gènes responsables des caractères Lac et Rap ne sont pas portés par le même plasmide.

2) Curage plasmidique des souches donneuses.

Les souches S77 et S114 (toutes deux L. lactis ssp cremoris) sont insensibles au curage à la novobiocine.
Les souches S91, S96 et S94 peuvent perdre par curage - semble-t-il de façon partielle - la capacité à fermenter le lactose (ce caractère n'est pas complètement perdu car après 24 h d'incubation à +30°C les clones Lac$^-$ développent une teinte jaune pâle sur milieu Elli-1 : il subsiste donc une activité Lac$^+$ résiduelle chez ces souches) mais elles conservent leur résistance aux phages.

3) Extraction plasmidique des souches donneuses et des transconjugants et analyse par électrophorèse.

Les extractions plasmidiques et analyse sur gel d'électrophorèse effectués selon la technique décrite en A)5), n'ont pas permis de visualiser les plasmides responsables des caractères Lac et Rap (ces résultats s'expliquent par la grande taille de ces plasmides, lesquels ne peuvent être analysés que par la technique de champ pulsé exposée dans l'exemple 4).

4) Transfert par conjugaison de la capacité de fermenter le lactose au deuxième cycle de conjugaison.

Le tableau 5 ci-après précise l'efficacité théorique Et de transfert de la capacité à fermenter le lactose, définie comme le rapport entre le nombre de transconjugants obtenus et le nombre initial de réceptrices, ainsi que l'efficacité réelle Er de transfert de la capacité à fermenter le lactose, égale à l'efficacité théorique multipliée par l'inverse du facteur de multiplication des réceptrices pendant la conjugaison, pour 5 clones de transconjugants Lac$^+$ et Rap$^+$. Le témoin est constitué par la souche S124 seule.

## Tableau 5

Efficacités théoriques et réelles du transfert par conjugaison de la capacité de fermenter le lactose.

| Souche donneuse | Et | Er |
|---|---|---|
| S45-77 clone 6 | 1 | $2 \times 10^{-1}$ |
| S45-91 clone 1 | $2,4 \times 10^{-1}$ | $2,3 \times 10^{-2}$ |
| S45-96 clone 1 | $7,3 \times 10^{-1}$ | $7 \times 10^{-2}$ |
| S45-94 clone 1 | $6,1 \times 10^{-7}$ | $5,7 \times 10^{-8}$ |

## Tableau 5 (suite)

| Souche donneuse | Et | Er |
|---|---|---|
| S45-114 clone 1 | $3,8 \times 10^{-7}$ | $4 \times 10^{-8}$ |
| S 124 | $<8 \times 10^{9}$ | |

On constate :

– La fréquence du transfert de la capacité de fermenter le lactose est considérablement plus importante avec ces transconjugants qu'avec les souches donneuses du premier cycle de conjugaison, résultat particulièrement net avec les transconjugants S45-77 clone 6, S45-91 clone 1 et S45-96 clone 1. On a par ailleurs vérifié sur 20 clones de ces transconjugants la résistance aux phages 059 (ainsi que la sensibilité à la streptomycine caractère porté par la souche réceptrice S124). Les caractères Rap et Lac sont donc transférés simultanément. Ces résultats étayent l'hypothèse (cf. notamment exemple 2 ci-dessus) selon laquelle le premier cycle de conjugaison a sélectionné un événement relativement rare : la formation d'un cointégrat entre deux plasmides, l'un porteur de la capacité de fermenter le lactose et l'autre d'un mécanisme de résistance aux phages. Le cointégrat étant formé, son transfert par conjugaison est plus fréquent, d'où une plus grande efficacité de transfert au deuxième cycle de conjugaison.

– L'efficacité réelle du transfert de ces caractères est comprise entre environ 2 et 20% pour les transconjugants S45-77 clone 6, S45-91 clone 1 et S45-96 clone 1.

Exemple 4 :

Isolement et caractérisation des plasmides portant des mécanismes de résistance aux phages.

A) Principales techniques expérimentales utilisées.

1) Electrophorèses en champ pulsé.

Elles ont été réalisées grâce au système "Pulsaphor Plus" (LKB-Pharmacia). Les gels sont constitués d'agarose 1%, 110 ou 130 ml selon les cas. La cuve contient 2,6 l de tampon TBE (composition : Tris HCl 30 mM pH 8,5, acide borique 50 mM, EDTA 1 mM). La migration s'effectue à 300 V constants, avec un temps de pulsation et une durée de migration déterminés à l'avance selon la séparation désirée des fragments d'ADN.

2) Préparation de marqueurs de taille.

Les oligomères d'ADN de phage Lambda ont été préparés comme cela est décrit par Waterbury et Lane (WATERBURY P.G. et LANE M.G. (1987) Nucleic Acids Res., 15, 3930).

Un stock de phages lambda purifiés sur chlorure de césium est dosé à 260 nm. Considérant qu'une $DO_{260}$ de valeur 1 correspond à 50 µg/ml d'ADN, 75 µg d'ADN, ramenés à 500 µl de tampon TMN (Tris-HCl 50 mM pH 8, $MgCl_2$ 10 mM, NaCl 10 mM), préchauffé à 37°C, sont mélangés à 500 µl d'agarose à bas point de fusion à 1,5% dans du tampon TMN maintenu à 45°C ; le mélange est immédiatement réparti dans des matrices en plastique de 125 µl prévues à cet effet, et refroidi à +4°C pendant 15 min. Les "inserts" ainsi constitués sont suspendus dans une solution composée de : EDTA 0,5 M, SDS 1%, protéinase K 1 mg/ml, Tris-HCl 10 mM pH 9, pendant 4 h à 55°C avec une faible agitation, les "inserts" sont ensuite dialysés contre 50 ml de Tris-HCl 10 mM pH 8, EDTA 10 mM, 2 x 1 h à +25°C.

3) Préparation des ADN genomiques.

Ils ont été préparés non par la méthode classique de Mc Clelland et al (Mc CLELLAND et al (1987) Nucleic Acids Res. 15, 5985-6005) mais par une technique dérivée de cette dernière exposée ci-après, permettant d'inclure 10 fois plus d'ADN génomique dans un volume donné d'agarose à bas point de fusion que cette méthode :

Une culture de nuit des souches en présence ou non d'antibiotiques et/ou de glucose à 0,4% est effectuée. Cette culture sert à ensemencer 15 ml de milieu de culture M17 à raison de 2% (volume/volume). L'incubation est réalisée à l'étuve jusqu'à ce qu'il y ait une densité optique de 0,5 à 0,8 à 600 nm. Puis la culture est centrifugée. Le culot est lavé 1 fois avec 5 ml de tampon SMC (saccharose 0,5 M ; maléate 0,02 M ; chlorure de calcium 0,05 M ; pH 6,5). Les cellules sont centrifugées et le culot est repris par 15 ml de tampon SMC contenant du lysozyme à 4 mg/ml pendant 2 h à 37°C.

Les cellules sont centrifugées et le culot est repris par 450 µl de tampon SMC. Les cellules sont incubées pendant 10 min à 37°C puis 500 µl sont prélevés et mélangés à 500 µl d'agarose à bas point de fusion à 2% dans du tampon SMC, maintenu en surfusion à 50°C. Immédiatement, cette solution est répartie dans des matrices en plastique prévues à cet effet, par partie aliquote de 125 µl. L'agarose se solidifie pendant 15 min à +4°C. Les "inserts" ainsi obtenus sont placés dans 2,5 ml de tampon de lyse (Tris-CHl 10 mM pH 9, EDTA 100 mM, EGTA 10 mM, 1% SDS, protéinase K 1 mg/ml) pendant 24 h à 55°C avec une faible agitation. Les "inserts" sont ensuite dialysés dans 50 ml de tampon (Tris-HCl 10 mM pH 8 ; EDTA 10 mM) avec 250 µl de PMSF (Phénylméthylsulfonylfluoride) à 20 mM pendant 1 h, à +25°C. Deux autres dialyses sont ensuite réalisées dans les mêmes conditions, mais sans PMSF. Les "inserts" peuvent aussi être ainsi conservés au moins 6 mois à +4°C dans du TE (Tris-CHl 10 mM pH 8 ; EDTA 10 mM).

La méthode de Mc Clelland et al (1987) consiste, pour sa part, à inclure les cellules en phase exponentielle de croissance dans de l'agarose à faible point de fusion et ensuite de faire le traitement au lysosyme puis à la protéinase K et au SDS. Le traitement des cellules au lysozyme avant leur inclusion dans l'agar permet d'augmenter la concentration d'ADN dans l'agarose.

4) Hydrolyse de l'ADN par une enzyme de restriction.

Un tiers ou un quart d'"insert" est placé dans un tube Eppendorf en présence de 1 ml de tampon d'hydrolyse préconisé par le fabricant. Les "inserts" sont incubés 15 min à 37°C en bain-marie. Puis 800 à 850 µl de tampon sont prélevés selon la taille de l'"insert" et 25 unités d'enzyme de restriction sont rajoutés au tampon restant. Les "inserts" sont incubés 24 h à la température d'activité optimale de l'enzyme.

5) Profils de restriction de plasmide en champ pulsé.

Il est nécessaire d'utiliser des "inserts" préparés selon la technique qui permet d'avoir 10 fois plus d'ADN (voir 3) ci-dessus). Après hydrolyse de l'ADN et migration en champ pulsé, les fragments d'ADN sont prélevés à partir du gel d'agarose. Ces morceaux d'agarose sont ensuite traités par des enzymes de restriction comme un "insert" classique.

6) Mise en évidence des plasmides sous forme circulaire en champ pulsé.

L'ADN hydrolysé par l'enzyme de restriction Apal d'une souche bactérienne migre en champ pulsé. Le gel est décollé et l'ADN subit une seconde migration en champ continu à 90° de la direction initiale après irradiation aux ultra-violets pendant 5 min. Cette technique, dérivée d'une méthode classique (Hintermann et col., (1981) Plasmid 5, 371-373) permet, d'une part de repérer l'ADN sous forme superenroulée qui migre différemment de l'ADN linéaire, d'autre part de libérer du plasmide sous forme circulaire relâchée.

B) Résultats.

1) Analyse des ADN génomiques des transconjugants curés et non curés :

L'intérêt principal de la technique d'électrophorèse sur gel d'agarose en champ pulsé est de permettre la séparation de fragments d'ADN de grande taille, en particulier d'une taille supérieure à 20 kb.

Les ADN totaux des transconjugants S45-91 clone 1, S45-96 clone 1, S45-77 clone 6, S45-114 clone 1 et S45-94 clone 1 ainsi que de la souche réceptrice (témoin), curés et non curés ont été hydrolysés à l'aide de l'enzyme de restriction Apal et les fragments de grande taille obtenus ont été analysés par électrophorèse en champ pulsé sur gel d'agarose. On a ainsi mis en évidence des fragments surnuméraires, présents dans les transconjugants et non dans la souche réceptrice.

Le tableau 6 ci-après précise pour les transconjugants la taille approximative (en kb) de ces fragments surnuméraires :

## Tableau 6

Corrélation entre les phénotypes obtenus avant et après curage à la novobiocine et les fragments surnuméraires visibles après migration en champ pulsé.

| Transconjugant | Phénotype | Fragment surnuméraire (kb) |
|---|---|---|
| S45-91 clone 1 non curé | Rap$^+$ Lac$^+$ | 144 |
| curé | Rap$^-$ Lac$^-$ | 0 |
| S45-96 clone 1 non curé | Rap$^+$ Lac$^+$ | 107 |
| S45-77 clone 6 non curé | Rap$^+$ Lac$^+$ | 118 |
| curé | Rap$^-$ Lac$^-$ | 0 |
| S45-114 clone 1 | | |
| non curé | Rap$^+$ Lac$^+$ | 66 |
| curé | Rap$^-$ Lac$^-$ | 0 |
| curé | Rap$^+$ Lac$^-$ | 66 |
| curé | Rap$^-$ Lac$^+$ | 0 |
| S45-94 clone 1 non curé | Rap$^+$ Lac$^+$ | 57 + 72 |
| curé | Rap$^-$ Lac$^-$ | 0 |
| curé | Rap$^+$ Lac$^-$ | 72 |

Rap : résistance aux phages ; Lac : capacité de fermenter le lactose.

On constate :

– les transconjugants S45-91 clone 1 et S45-77 clone 6 présentent le phénotype Rap$^+$ Lac$^+$ en présence d'un fragment surnuméraire de 144 kb et (respectivement) d'un fragment surnuméraire de 118 kb. Après curage ils présentent le phénotype Rap$^-$ Lac$^-$ en l'absence de fragment surnuméraire. Les caractères Lac et Rap sont donc portés par chacun de deux fragments surnuméraires ci-dessus.

– Le transconjugant S45-96 clone 1 ne présente que le phénotype Rap$^+$ Lac$^+$ lié à la présence d'un fragment surnuméraire de 107 kb, qu'il n'est pas possible de curer à la novobiocine. Celui-ci porte donc les caractères Rap et Lac.

– Le transconjugant S45-114 clone 1 Rap$^+$ Lac$^+$ présente un fragment surnuméraire de 66 kb. On peut corréler la disparition du phénotype Rap$^+$ après curage à celle de ce fragment surnuméraire. Celui-ci porte donc un mécanisme de résistance aux phages (le caractère Lac est probablement porté par un autre fragment surnuméraire non détecté).

– Le transconjugant S45-94 clone 1 Rap$^+$ Lac$^+$ présente deux fragments surnuméraires de 57 et 72 kb. On peut corréler la disparition du phénotype Rap$^+$ après curage à celle du fragment surnuméraire de 72 kb (et celle du phénotype Lac$^+$ à celle du fragment surnuméraire de 57 kb).

Le fragment surnuméraire de 72 kb porte donc un mécanisme de résistance aux phages (et le fragment surnuméraire de 57 kb le caractère Lac).

Les fragments surnuméraires de 144 kb, 107 kb, 118 kb, 66 kb et 72 kb sont donc porteurs d'un mécanisme de résistance aux phages. Par la suite ils seront appelés respectivement pPF144, pPF107, pPF118, pPF66 et pPF72.

2) Caractérisation des fragments d'ADN surnuméraires :

Les fragments surnuméraires pPF144, pPF107, pPF118, pPF66 et pPF72, ont été hydrolysés par l'enzyme de restriction Apal et soumis au traitement décrit en A)6). Cette expérience a permis de montrer qu'ils sont tous linéaires dans les morceaux de gels d'agarose prélevés.

a) Cartes de restriction des fragments surnuméraires pPF144, pPF107, pPF66 et pPF72 :

Afin d'établir leurs cartes de restriction, les fragments surnuméraires pPF144, pPF107, pPF66 et pPF72 ont été soumis à des digestions simples, doubles ou triples à l'aide de différentes enzymes de restriction, notamment les enzymes Apal, BamHI, BglI, SacI, SacII et SalI et les fragments obtenus ont été analysés sur gel d'agarose en champ pulsé. Quand cela a été possible, l'ADN génomique des transconjugants a d'autre part été digéré à l'aide d'autres enzymes que Apal afin de linéariser les fragments surnuméraires à un autre endroit, et analysé par électrophorèse en champ pulsé. Ceci a permis de confirmer les cartes de restriction et de démontrer pour les fragments surnuméraires pPF107, pPF144 et pPF66 le caractère circulaire initial. Ceux-ci sont donc des plasmides.

Les tableaux 8, 9, 10 et 11 ci-après précisent les profils de restriction obtenus pour les fragments pPF144, pPF107, pPF66 et pPF72.

## Tableau 8

Fragments de restriction obtenus avec le plasmide pPF144.

| Enzymes de restriction | Nombre de fragments | Taille en kb |
|------------------------|---------------------|--------------|
| ApaI BamHI | 2 | 86, 58 |
| ApaI BglI | 3 | 50, 50, 44 |
| ApaI SalI | 2 | 79, 65 |
| ApaI SacII | 2 | 86, 58 |
| ApaI SmaI | 2 | 127, 17 |

## Tableau 8 (suite)

| Enzymes de restriction | Nombre de fragments | Taille en kb |
|---|---|---|
| SacII BgII | 3 | 94, 36, 14 |
| SacII SaII | 2 | 137, 7 |
| SacII ApaI | 2 | 86, 58 |
| SacII SmaI | 2 | 103, 41 |
| SacII BamHI | 1 | 144 |

(La première enzyme (Apal ou SacII) est celle utilisée pour linéariser le plasmide pPF144, lors de la digestion de l'ADN total du transconjugant. La seconde enzyme a été utilisée pour digérer le fragment obtenu lors de la digestion avec la première enzyme).

## Tableau 9

Fragments de restriction obtenus avec le plasmide pPF107.

| Enzymes de restriction | Nombre de fragments | Taille en kb |
|---|---|---|
| ApaI SaII | 3 | 64, 27, 16 |
| ApaI BamHI | 3 | 71, 27, 9 |
| ApaI BgII | 3 | 77, 27, 3 |
| ApaI SmaI | 2 | 91, 16 |
| ApaI SacII | 3 | 80, 17, 10 |

Tableau 9 (suite)

| Enzymes de restriction | Nombre de fragments | Taille en kb |
|---|---|---|
| BgII SaII | 2 | 61, 43 |
| BgII BamHI | 2 | 98, 6 |
| BgII ApaI | 2 | 77, 27 |
| BgII SmaI | 2 | 61, 43 |
| BgII SacII | 2 | 87, 17 |

(La première enzyme (ApaI ou BgII) est celle utilisée pour linéariser le plasmide pPF107, lors de la digestion de l'ADN total du transconjugant. La seconde a été utilisée pour digérer le fragment obtenu lors de la digestion avec la première enzyme).

Tableau 10

Fragments de restriction obtenus avec le plasmide pPF66.

| Enzymes de restriction | Nombre de fragments | Taille en kb |
|---|---|---|
| ApaI SaII | 4 | 31, 26, 7, 2 |
| ApaI BamHI | 2 | 45, 21 |
| ApaI BgII | 2 | 33, 33 |
| BamHI SaII | 4 | 26, 19, 12, 9 |
| BamHI ApaI | 2 | 45, 21 |
| BamHI BgII | 2 | 54, 12 |

La première enzyme (ApaI ou BamHI) est celle utilisée pour linéariser le plasmide pPF66, lors de la digestion de l'ADN total du transconjugant. La seconde enzyme a été utilisée pour digérer le fragment obtenu après la digestion par la première enzyme).

## Tableau 11

Fragments de restriction obtenus avec le plasmide pPF72.

| Enzyme de restriction | Nombre de fragments | Taille en kb |
|---|---|---|
| ApaI SaII | 5 ou 4 | 31, 26, 11, 2, 2 |
| ApaI BamHI | 3 | 42, 21, 9 |
| ApaI SacI | 2 | 45, 27 |
| ApaI SacII | 2 | 62,5, 9,5 |

Ces profils de restriction ont permis notamment d'établir des cartes de restriction des fragments pPF144, ppF107, pPF66 et pPD72, représentées respectivement sur les figures 1, 2, 3 et 4, les chiffres vis-à-vis des sites de restriction symbolisant le nombre de kb à partir du site ApaI, pris arbitrairement comme origine. Sur ces figures sont représentés au même endroit les sites que la sensibilité de la méthode d'analyse n'a pas permis de séparer.

b) Profil de restriction du fragment surnuméraire pPF118.

Le fragment surnuméraire pPF118 déjà digéré par ApaI a été soumis à une digestion simple à l'aide des enzymes BamHI, XhoI, BgII et SaII. Le tableau 12 ci-après rassemble les profils de restriction obtenus.

## Tableau 12

Taille des fragments (kb) obtenus par digestion du fragment pPF118 (déjà digéré par ApaI).

pPF118

| BamHI | XhoI | BgII | SaII |
|---|---|---|---|
| 66 | 95 | 71 | 46 |
| 36 | 26 | 28 | 42 |
| 16 | | 17 | 36 |
| | | 16 | 23 |
| | | 15 | |

c) Caractère plasmidique des fragments pPF72 et pPF118 :

Il a été possible de curer les souches S45-94 clone 1 et S45-77 clone 6 des fragments pPF72 et pPF118 (cf. ci-dessus tableau 3 de l'exemple 2), ce qui montre le caractère extra chromosomique de ces fragments.

On a d'autre part constaté par analyse à l'aide de la technique d'électrophorèse en champ pulsé l'absence de fragment linéaire dans l'ADN génomique non digéré de ces souches et la présence des fragments linéaires pPF72 et pPF118 dans l'ADN génomique de ces souches digéré par Apal. Ces deux fragments extra chromosomiques sont donc sous forme circulaire dans les souches S45-94 clone 1 et S45-77 clone 6.

Les fragments surnuméraires pPF72 et pPF118 sont donc également des plasmides.

d) Positionnement du gène de la P-β-galactosidase sur les plasmides pPF144 et pPF107 - Présence de ce gène sur le plasmide pPF118 et absence de celui-là sur les plasmides pPF66 et pPF72.

Le gène de la P-β-galactosidase, enzyme impliquée dans le métabolisme du lactose, doit être présent sur les plasmides pPF144 et pPF107, porteurs du caractère Lac. Ce gène a été cloné par B. BOIZET et al dans le plasmide pUCB25 (B. BOIZET et al (1988) Gene, 62, 249-261). Ce dernier a été utilisé comme sonde pour positionner par hybridation le gène de la P-β-galactosidase dans les plasmides ci-dessus.

On a ainsi montré que le gène de la P-β-galactosidase est situé sur les plasmides pPF114 et pPF107 dans la partie qui comprend le site Apal. Ce résultat n'est pas étonnant car la séquence publiée de ce gène (B. BOIZET et al (1988) Gene, 62, 249-261) comprend un site Apal.

L'application de la technique décrite ci-dessus au fragment pPF118 et aux plasmides pPF66 ainsi que pPF72 a d'autre part permis de vérifier la présence du gène de la β-galactosidase dans le plasmide pPF118 et son absence dans les plasmides pPF66 et pPF72.

Exemple 5 :

Caractérisation partielle des mécanismes de résistance aux phages - Thermosensibilité - Etude de l'adsorption phagique.

1) Thermosensibilité des mécanismes de résistance :

Afin de déterminer la résistance à la température de ces différents mécanismes de résistance aux phages, le test suivant a été effectué sur les transconjugants :

Les bactéries à tester sont cultivées jusqu'à une phase exponentielle de croissance ($DO_{600}$ = 0,5). Puis ces bactéries sont préchauffées à la température que l'on veut tester pendant 15 min de même que les boîtes de milieu gélosé. Ensuite un tapis de ces bactéries est coulé avec de la gélose molle contenant du $CaCl_2$ O 10 mM et des gouttes de phages à différentes dilutions sont déposées sur ce tapis. La boîte Pétri est ensuite immédiatement mise à la température testée, dans une étuve appropriée.

Le tableau 13 ci-après rassemble les résultats obtenus pour les titres phagiques pour les 5 transconjugants retenus en 1) et la souche S45 (témoin) à une température de 30°C et de 40°C.

## Tableau 13

Résistance aux phages des transconjugants Lac$^+$ aux températures de 30°C et 40°C

| Souche | Phage [30°C] Titre phagique [nombre de phages/ml]/ diamètre de la plage de lyse [mm] | | | Phage [40°C] Titre phagique [nombre de phages/ml]/ diamètre de la plage de lyse [mm] | | |
|---|---|---|---|---|---|---|
| | 024 [groupe I] | 053 [groupe I] | 059 [groupe III] | 024 [groupe I] | 053 [groupe I] | 059 [groupe III] |
| S45-91 clone 1 | 0/0 | $4,10^7/<0,25$ | 0/0 | $6,10^6/<0,25$ | $2,10^8/<0,25$ | 0/0 |
| S45-96 clone 1 | 0/0 | 0/0 | 0/0 | $9,10^8/2$ | $2,10^{10}/3$ | $4,10^8/2$ |
| S45-77 clone 6 | $8,10^7/<0,25$ | $2,10^{10}/<0,25$ | 0/0 | $2,10^9/<0,25$ | $2,10^{10}/<0,25$ | $4,10^8<0,25$ |
| S45-114 clone 1 | $8,10^6/<0,25$ | $2,10^9/<0,25$ | 0/0 | $9,10^8/<0,25$ | $4,10^8/<0,25$ | $5,10^7/<0,25$ |
| S45-94 clone 1 | 0/0 | $10^{10}/<0,25$ | 0/0 | 0/0 | $2,10^8<0,25$ | 0/0 |
| S45 | $2,10^9/2$ | $2,10^{10}/3$ | $3,10^9/2$ | $2,10^9/2$ | $2,10^{10}/3$ | $2,10^9/2$ |

EP 0 452 224 A1

On constate à la lecture du tableau ci-dessus :

– la souche S45-96 clone 1, totalement résistante aux phages à la température de 30°C devient pratiquement aussi sensible que la souche témoin à une température de 40°C. Le mécanisme de résistance aux phages mis en jeu est donc très thermosensible.

– Les souches S45-91 clone 1, S45-77 clone 6 et S45-114 clone 1 ont une résistance (partielle) aux phages à une température de 40°C inférieure à celle qu'elles ont à une température de 30°C. Les mécanismes de résistances aux phages portés par ces souches sont donc moyennement thermosensibles.

– La souche S45-94 clone 1 est aussi résistante à une température de 40°C qu'à une température de 30°C. Son mécanisme de résistance aux phages est donc peu thermosensible.

Les résultats rapportés ci-dessus laissent supposer que différents types de mécanismes, certains plus thermosensibles que d'autres, sont impliqués dans la résistance aux phages.

### 2) Etude de l'adsorption phagique.

On se place dans des conditions où la bactérie provient d'une culture de nuit. Puis dans un tube Eppendorf sont déposés :
- 600 μl de culture bactérienne ;
- 30 μl de CaCl$_2$, 1M ;
- 600 μl de phages (à 10$^5$/ml).

L'ensemble est laissé à l'étuve à 30°C pendant exactement 12 min, puis l'adsorption est arrêtée en plongeant le tube Eppendorf dans la glace. Le tube est centrifugé dans une microfuge à tubes Eppendorf pendant 5 min à +4°C pour se débarrasser des bactéries et des phages adsorbés. Le surnageant est prélevé, dilué à 4°C ; le nombre de phages non adsorbés est ainsi déterminé sur des tapis de la souche S45.

Ce test a été effectué sur les 5 transconjugants retenus et la souche S45 (témoin) avec comme phages 053 (groupe I d'homologie), phage contre lequel les transconjugants sont partiellement résistants, et avec 059 (groupe III d'homologie), phage contre lequel les transconjugants sont résistants (cf. tableau 2 ci-dessus).

Aucune différence d'adsorption phagique significative n'a été constatée, contrairement aux observations de SANDERS M.E. et al et de DE VOS W.M. et al (SANDERS M.E. et al (1983), Appl. Environ Microbiol. 46, 1125-1133 et DE VOS W.M. et al (1984), Microbiol. Lett., 23, 175-178).

L'adsorption phagique n'est donc pas impliquée dans les mécanismes de résistance portés par ces souches.

### Exemple 6:

### Recherche d'activité bactéricide

Il est intéressant d'avoir des souches de lactocoques possédant une activité bactéricide (provoquée par des bactériocines) car celles-ci inhibent le développement de bactéries contaminantes. On a donc recherché des activités bactéricides dans les transconjugants retenus.

### a) Protocole.

7 μl d'une culture de nuit de la souche étudiée sont déposés sur une boîte de milieu gélosé sous forme d'une goutte. L'incubation dure 16 h à la température optimale de croissance. Puis sur ce milieu est étalée la souche bactérienne sensible, Lactococcus lactis ssp lactis S45, mélangée à de la gélose molle à 50°C, du milieu et du glucose à 0,4%. L'incubation est effectuée de nouveau pendant 16 h. L'apparition d'un halo clair autour de la goutte de culture laisse présumer la présence d'une bactériocine (Van BELKUM et al (1989) Appl. Environ. Microbiol. 55, 1187-1191).

### b) Résultats :

Le protocole décrit ci-dessus a été mis en oeuvre pour les souches ci-après :
- les transconjugants (non curés) S45-91 clone 1, S45-96 clone 1, S45-77 clone 6, S45-114 clone 1, S45-94 clone 1 ;
- le transconjugant curé S45-114 clone 1 présentant les phénotypes Rap$^+$ Lac$^-$, Rap$^-$ Lac$^-$ et Rap$^-$ Lac$^+$ et le transconjugant curé S45-94 clone 1 présentant les phénotypes Rap$^-$ Lac$^-$ et Rap$^+$ Lac$^-$ ;
- les souches donneuses S91, S94, S96, S114 et S77 ;
- la souche réceptrice S45 (témoin).

Les souches ci-après présentent une activité bactéricide : les souches donneuses S94 et S114, les transconjugants S45-94 clone 1 et S45-114 clone 1, le transconjugant curé S45-94 clone 1 présentant le phénotype Rap$^+$ Lac$^-$ et le transconjugant curé S45-114 clone 1 présentant le phénotype Rap$^+$ Lac$^-$. Ces transconjugants curés ou non contiennent tous l'un ou l'autre des plasmides pPF72 et pPF66. Chacun de ceux-ci possède donc une activité bactéricide.

Des expériences complémentaires ont montré que les souches possédant le plasmide pPF66 sont résistantes à l'activité bactéricide des souches possédant le plasmide pPF72 et vice versa. Or on sait que les bactéries sont résistantes aux bactériocines qu'elles produisent. Les modes d'action des mécanismes de résistance aux bactériocines sont donc voisins.

## Exemple 7:

### Clonage d'un fragment du plasmide pPF144 d'environ 3,3 kb porteur d'un ou plusieurs mécanismes de résistance aux phages

1) Clonage d'un fragment du plasmide pPF144 d'environ 3,3 kb, supposé porteur d'un ou plusieurs mécanismes de résistance aux phages

L'ADN du transconjugant S45-91 clone 1 (déposé à la C.N.C.M. sous le n° I-945) a été extrait, hydrolysé par l'endonucléase Apal et soumis à une électrophorèse en champ pulsé dans les conditions décrites dans l'exemple 4. Puis l'ADN linéarisé du plasmide pPF144 a été extrait à partir du gel d'électrophorèse obtenu, à l'aide du kit "geneclean" (Bio 101-La Jolla-Californie, Etats-Unis). La technique d'extraction utilisée dans ce kit consiste à dissoudre l'agarose avec une solution d'iodure de sodium, adsorber l'ADN avec une suspension de microparticules de silice, appelée lait de silice, centrifuger cette suspension en éliminant le surnageant et remettre en solution l'ADN à l'aide d'une solution Tris : 10 mM, EDTA : 10 mM, pH8. Cet ADN a ensuite été hydrolysé par les enzymes de restriction EcoRI et HindIII, enzymes dont on a au préalable constaté que chacune coupait le plasmide pPF144 en un grand nombre de fragments. L'ensemble des différents fragments d'ADN obtenu est appelé mélange A.

Par ailleurs, le plasmide navette pVA838, déjà mentionné, qui porte une origine de réplication chez E. coli, notée oric, et une origine de réplication chez les lactocoques, notée oris, ainsi qu'un gène de résistance à l'érythromycine, noté Ery$^R$, a été hydrolysé par ces deux enzymes, EcoRI et HindIII, libérant ainsi 3 fragments : un fragment HindIII-HindIII d'environ 4,7 kb, appelé fragment B, contenant oris et Ery$^R$, un fragment EcoRI-HindIII d'environ 1,5 kb, appelé fragment C contenant oric et un fragment EcoRI-HindIII d'environ 2 kb. Ces 3 fragments ont été séparés sur gel d'électrophorèse, et seulement deux d'entre eux, les fragments B et C, ont été prélevés. La purification de ces fragments a été effectuée avec le kit "geneclean". Ces deux fragments B et C ne peuvent se liguer de façon à fournir un plasmide fonctionnel car les extrémités EcoRI et HindIII ne sont pas compatibles. Pour redonner un plasmide fonctionnel, il faut que ces deux fragments s'associent à un troisième fragment aux extrémités EcoRI et HindIII : cette approche est une stratégie de clonage orientée : seuls les plasmides possédant un nouveau fragment EcoRI-HindIII, seront sélectionnés.

Les fragments B et C, ainsi que le mélange A, sont mis en contact : on effectue une ligation de l'ensemble à l'aide de la DNA-ligase, puis une transformation par électroporation de la souche de E. coli DB11 (sélection sur milieu L + erythromycine à 25 μg/ml). 3400 clones sont obtenus.

Après extraction de l'ADN plasmidique de 20 clones, et digestion par EcoRI et HindIII, 18 d'entre eux possèdent les fragments B et C et un troisième fragment visible sur gel d'agarose à 1 %. Les 3400 clones ont donc été mélangés, l'ADN plasmidique total a été extrait et a servi à transformer la souche Lactococus lactis ssp lactis S45 curée du plasmide pVA838, appelée souche S45d, selon la technique de transformation par formation et régénération de protoplastes décrite par Von Wright et al, 1985, App. Env. Microbiol, 50, 1100-1102. Le protocole utilisé est exposé ci-après.

Protocole de transformation des Lactocoques par formation et régénération de protoplastes

100 μl d'une culture de nuit servent à ensemencer 5 ml de milieu M17 glucosé à 4 g/l et l'ensemble est placé à l'étuve jusqu'à une densité optique à 600 nm de 0,5. Les cellules sont centrifugées et mises en suspension dans 5 ml de tampon appelé SMC de composition : saccharose 0,5 M ; maléate 20 mM ; CaCl2 50 mM pH 6,5, additionné de 4 mg/ml de lysozyme. L'incubation est effectuée pendant deux heures à 37 °C puis les cellules sont centrifugées à 25 °C, lavées dans du tampon SMC, centrifugées de nouveau et mises en suspension dans 500 μl de tampon SMC : ceci constitue la préparation de protoplastes. Ensuite, sont ajoutés dans cet ordre dans un tube Eppendorf stérile et mélangés à chaque fois :

– 5 μl de tampon SMC pH 6,5 (2 fois)

- 5 µl préparation d'ADN dans de l'eau déminéralisée
- 100 µl de protoplastes
- 330 µl d'une solution de polyéthylène glycol de poids moléculaire moyen 6000, à 30 % dans le tampon SMC

L'ensemble est incubé pendant 10 min à 25°C puis on ajoute 1 ml de tampon SMC pour arrêter l'action du PEG (en mélangeant doucement). Les cellules sont centrifugées. Le surnageant est jeté et 1 ml de milieu M17 (décrit précédemment) additionné de 0,5 M de saccharose de 4 g/l de glucose est ajouté. Une expression est réalisée pendant 1 h à 30°C. Les cellules sont centrifugées. Le surnageant est presque entièrement jeté, le culot est mis en suspension dans le liquide résiduel et mélangé avec de l'agar mou en surfusion contenant du glucose à 4 g/l, 5 µl/ml d'érythromycine et du saccharose 0,5 M ; ce mélange est étalé sur des boîtes de M17 gélosé (décrit précédemment) additionné de 0,5 M de saccharose et de 4 g/l de glucose. Les boîtes sont placées à l'étuve en anaérobiose pendant 4 à 5 jours. Les clones commencent à apparaître au bout de deux à trois jours d'incubation.

215 clones ont ainsi été obtenus : ils ont tous été testés pour leur résistance au phage ∅59 selon la technique décrite précédemment (cf. exemple 4). Un seul clone est résistant à ce phage. La résistance aux phages de ce clone est identique à celle observée chez le transconjugant S45-91 clone 1 : résistance totale au phage ∅59 du groupe III et résistance partielle au phage ∅53 du groupe I. Après extraction du plasmide contenu dans cette souche, appelé plasmide pPF 144-1, sa carte de restriction a été établie. Elle est représentée sur la figure 5, les chiffres en vis-à-vis des sites de restriction symbolisant le nombre de kb à partir d'un site HindIII, pris arbitrairement comme origine. On constate la présence des fragments B et C provenant du vecteur pVA838 d'un fragment HindIII-HindIII d'environ 3,3 kb, appelé fragment D, supposé porteur d'un ou plusieurs mécanismes de résistance aux phages, ce caractère étant noté Rap, ainsi que d'un fragment EcoRI-HindIII d'environ 0,2 kb, appelé fragment E, les fragments D et E provenant du mélange A.

2) Vérification que le fragment D est porteur d'un ou plusieurs mécanismes de résistance aux phages

1. Pour contrôler que le plasmide pPF144-1 confère bien la résistance aux phages, ce plasmide a été extrait selon la technique décrite dans la section 4, puis a servi à transformer par électroporation la souche E. coli DB11. La souche transformée obtenue est appelée souche DB11 pPF144-1. Dans un deuxième temps, ce plasmide a été extrait de la souche DB11 pPF144-1 et a servi à transforme la souche S45d : il a alors été vérifié que la souche transformée présente la même résistance aux phages.

2. Afin de vérifier que le fragment D confère la résistance aux phages, le fragment D a été inséré dans le plasmide pVA838 de la façon suivante : le plasmide pVA838 et hydrolysé à l'aide de l'endonucléase HindIII ; on ajoute le fragment D aux deux fragments obtenus, on effectue une ligation à l'aide de la DNA-ligase et on transforme par électroporation la souche E. coli DB11 ; on extrait les plasmides de vingt clones. Seul l'un d'entre eux possède les deux fragments HindIII-HindIII de pVA838 et le fragment D. On a établi la carte de restriction du plasmide obtenu, appelé plasmide pPF144-2. Elle est représentée sur la figure 6. Après transformation de la souche S45d, il a été vérifié que la souche transformée, appelée souche S45d pPF144-2 est résistante aux phages, donc que le fragment D confère la résistance aux phages. Etant donné que le fragment est bordé par 2 sites HindIII, son orientation n'est pas connue dans les plasmides pPF144-1 et pPF144-2.

La souche DB11 pPF144-1 a été déposée à la C.N.C.M. sous le n° I-1070 le 09 Avril 1991.

3) hybridation avec le fragment D

L'ADN de chacun des transconjugants obtenus a été extrait, hydrolysé par l'enzyme de restriction ApaI, puis soumis à une électrophorèse en champ pulsé comme décrit dans la section 4. Après dénaturation à l'aide de soude de cet ADN et séchage du gel, on a effectué l'hybridation avec le fragment D dans les conditions classiques de Southern Blot (Maniatis, op cité). Il a ainsi été constaté que chacun des plasmides pPF144, pPF107, pPF118, pPF72 et pPF66 s'hybride avec le fragment D (le signal obtenu avec le plasmide pPF144 est beaucoup plus intense qu'avec les autres plasmides).

## Revendications

1. Molécule d'ADN comprenant au moins un mécanisme de résistance aux phages, caractérisée en ce qu'elle comporte une partie fonctionnelle du fragment HindIII-HindIII d'environ 3,3 kb du plasmide pPF144-1 présent dans la souche déposée à la C.N.C.M. sous le n° I-1070.

2. Plasmide comprenant au moins un mécanisme de résistance au phages, caractérisé en ce qu'il est une molécule selon la revendication 1.

3. Plasmide comprenant au moins un mécanisme de résistance aux phages, caractérisé en ce qu'il est contenu dans les transconjugants issus du croisement d'une souche donneuse choisie parmi les souches :
   – souches Lactococcus lactis ssp cremoris déposées à la C.N.C.M. sous les n° I-939 et I-943,
   – souche Lactococcus lactis ssp lactis déposée à la C.N.C.M. sous le n° I-940,
   – souches Lactococcus lactis ssp lactis var. diacetylous déposées à la C.N.C.M. sous les n° I-941 et I-942,
   et d'une souche réceptrice.

4. Plasmide selon la revendication 3, caractérisé en ce qu'il est susceptible de s'hybrider avec la molécule d'ADN selon la revendication 1.

5. Plasmide selon la revendication 4, caractérisé en ce que la souche réceptrice est la souche Lactococcus lactis ssp lactis S45.

6. Plasmide selon l'une des revendications 2 à 5, caractérisé en ce que le ou les mécanismes de résistance aux phages confèrent à une température de 30°C une résistance partielle ou totale contre les phages du groupe I d'homologie, et les phages du groupe III d'homologie.

7. Plasmide selon l'une des revendications 2 à 6, caractérisé en ce qu'il comprend également un ou plusieurs gènes conférant à la bactérie lactique le contenant une activité bactéricide.

8. Plasmide selon l'une des revendication 2 à 7, caractérisé en ce qu'il a les caractéristiques d'un plasmide choisi parmi :
   – le plasmide pPF66 contenu dans la souche Lactococcus lactis ssp lactis déposée à la C.N.C.M. sous le n° I-948,
   – le plasmide pPF72 contenu dans la souche Lactococcus lactis ssp lactis déposée à la C.N.C.M. sous le n° I-946,
   – le plasmide pPF107 contenu dans la souche Lactococcus lactis ssp lactis déposée à la C.N.C.M. sous le n° I-947,
   – le plasmide pPF118 contenu dans la souche Lactococcus lactis ssp lactis déposée à la C.N.C.M. sous le n° I-944,
   – le plasmide pPF144 contenu dans la souche Lactococcus lactis ssp lactis déposée à la C.N.C.M. sous le n° I-945.

9. Bactérie lactique résistante aux phages, caractérisée en ce qu'elle contient une molécule d'ADN selon la revendication 1.

10. Bactérie lactique résistante aux phages, caractérisée en ce qu'elle contient au moins un plasmide selon l'une des revendications 2 à 8.

11. Bactérie lactique selon l'une des revendications 9 et 10, caractérisée en ce qu'elle appartient à l'espèce Lactococcus lactis.

12. Bactérie lactique selon la revendication 11, caractérisée en ce qu'elle est choisie parmi les souches Lactococcus lactis ssp lactis déposée à la C.N.C.M. sous les n° I-944, I-945, I-946, I-947 et I-948.

13. Utilisation d'une souche choisie parmi les souches Lactococcus lactis ssp lactis déposées à la C.N.C.M. sous les n° I-944, I-945, I-946, I-947 et I-948, les souches Lactococcus lactis ssp cremoris déposées à la C.N.C.M. sous les n° I-939 et I-943, la souche Lactococcus lactis ssp lactis déposée à la C.N.C.M. sous le n° I-940, et les souches Lactococcus lactis ssp lactis var. diacetylous déposées à la C.N.C.M. sous les n° I-941 et I-942 pour conférer un mécanisme de résistance aux phages à une souche d'intérêt industriel.

14. Souche d'intérêt industriel résistante aux phages, caractérisée en ce qu'elle comporte un mécanisme de résistance aux phages transféré selon un procédé utilisant une souche choisie parmi les souches Lactococcus lactis ssp lactis déposées à la C.N.C.M. sous les n° I-944, I-945, I-946, I-947 et I-948, les souches Lactococcus lactis ssp cremoris déposées à la C.N.C.M. sous les n°I-939 et I-943, la souche Lactococcus lactis ssp lactis déposée à la C.N.C.M. sous le n° I-940 et les souches Lactococcus lactis ssp lactis var. diacetylous déposées à la C.N.C.M. sous les n° I-941 et I-942.

15. Utilisation d'une souche choisie parmi les souches <u>Lactococcus lactis ssp cremoris</u> déposées à la C.N.C.M. sous les n° I-939 et I-943, la souche <u>Lactococcus lactis ssp lactis</u> déposée à la C.N.C.M. sous le n° I-940 ainsi que les souches <u>Lactococcus lactis ssp lactis var. diacetylous</u> déposées à la C.N.C.M. sous les n° I-941 et I-942, pour obtenir un plasmide selon l'une des revendications 2 à 8.

# FIG.1

Plasmide pPF 144

# FIG. 2

Plasmide pPF 107

# FIG. 3

Plasmide pPF66

# FIG. 4

Plasmide pPF 72

# FIG.5

Plasmide pPF 144-1

# FIG.6

Plasmide pPF 144-2

Office européen
des brevets

**RAPPORT DE RECHERCHE EUROPEENNE**

Numero de la demande

EP    91 40 0982
Page 1

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| Y | EP-A-246909 (THE NATIONAL DEVELOPMENT CORPORATION LIMITED) <br> * le document en entier * <br> --- | 1-4, 9 | C12N15/74 <br> C12N1/20 <br> C12N1/21 <br> (C12N1/20, C12R <br> : 1/46) |
| Y | EP-A-208468 (NORTH CAROLINA STATE UNIVERSITY) <br> * le document en entier * <br> --- | 1-4, 9 | |
| X | APPLIED AND ENVIRONMENTAL MICROBIOLOGY <br> vol. 46, no. 5, 1983, <br> pages 1125 - 1133; SANDERS, M.E. et al.: <br> "Characterization of phage-sensitive mutants from a phage insensitive strain of Streptococcus lactis: Evidence for a plasmid determinant that prevents phage adsorption." <br> * le document en entier * <br> --- | 1-4, 9-11 | |
| X | APPLIED AND ENVIRONMENTAL MICROBIOLOGY <br> vol. 50, no. 4, octobre 1985, <br> pages 851 - 858; STEENSON, L.R. et al.: <br> "Streptococcus cremoris M12R transconjugants carrying the conjugal plasmidpTR2030 are insensitive to attack by lytic bacteriophages" <br> * le document en entier * <br> --- | 1-4, 9-11 | |
| X | APPLIED AND ENVIRONMENTAL MICROBIOLOGY <br> vol. 55, no. 6, juin 1989, <br> pages 1537 - 1543; JARVIS, A.W. et al.: <br> "Resistance against industrial bacteriophages conferred on Lactococci by plasmid pAJ1106 and related plasmids" <br> * le document en entier * <br> --- | 1-3, 9, 10 | |
| X | APPLIED AND ENVIRONMENTAL MICROBIOLOGY <br> vol. 55, no. 7, juin 1989, <br> pages 1684 - 1689; HILL, C. et al.: <br> "Localization cloning and expression of genetic determinants for bacteriophage resistance (Hsp) from the conjugative plasmid pTR2030" <br> * le document en entier * <br> --- | 1-3, 8, 9 | |

**DOMAINES TECHNIQUES RECHERCHES (Int. Cl.5)**

C12N
C12R

Le présent rapport a été établi pour toutes les revendications

| Lieu de la recherche | Date d'achèvement de la recherche | Examinateur |
|---|---|---|
| LA HAYE | 10 JUILLET 1991 | CHAMBONNET F.J. |

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

 

& : membre de la même famille, document correspondant

EPO FORM 1503 03.82 (P0402)

Office européen
des brevets

# RAPPORT DE RECHERCHE EUROPEENNE

Numero de la demande

EP    91 40 0982
Page 2

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5 ) |
|---|---|---|---|
| X | JOURNAL OF GENERAL MICROBIOLOGY vol. 131, no. 6, juin 1985, pages 1531 - 1542; KLAENHAMMER, T.R. et al.: "Conjugal tranfer from Streptococcus lactis ME2 of plasmids encoding phageresistance, nisin resistance and lactose fermenting ability: Evidence for high-frequency conjugative plasmid responsible for abortive i" * le document en entier * | 1-4, 6, 7, 9, 10 | |
| X | JOURNAL OF DAIRY SCIENCE. vol. 72, no. 12, 1989, CHAPAIGN, ILLINOIS U pages 3429 - 3443; KLAENHAMMER, T.R.: "Genetic characterization of multiple mechanisms of phage defense from a prototype phage-insensitive strain, Lactococcus lactis ME2" * le document en entier * | 1-4 | |
| X | NETHERLANDS MILK AND DAIRY JOURNAL. vol. 43, 1989, WAGENINGEN NL pages 245 - 259; VLEGELS, P.A.P. et al.: "Plasmid profiles of Lactococcus lactis subsp. cremoris strains and mutants in relation to phage resistance" * le document en entier * | 1-4, 9-11 | DOMAINES TECHNIQUES RECHERCHES (Int. Cl.5 ) |
| X | NETHERLANDS MILK AND DAIRY JOURNAL. vol. 43, 1989, WAGENINGEN NL pages 229 - 244; COFFEY, A.G. et al.: "Identification and cheracterisation of a plasmid encoding abortive infection from Lactococcus lactis ssp. lactis UC811" * le document en entier * | 1-4, 9-11 | |
| X | BIOCHIMIE vol. 70, 1988, PARIS FR pages 411 - 421; SANDERS, M.E.: "Phage resistance in lactic acid bacteria" * le document en entier * | 1, 3 | |

Le présent rapport a été établi pour toutes les revendications

| Lieu de la recherche | Date d'achèvement de la recherche | Examinateur |
|---|---|---|
| LA HAYE | 10 JUILLET 1991 | CHAMBONNET F.J. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)

Office européen
des brevets

# RAPPORT DE RECHERCHE EUROPEENNE

Numero de la demande

EP 91 40 0982
Page 3

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5 ) |
|---|---|---|---|
| X | JOURNAL OF DAIRY SCIENCE. vol. 71, 1988, CHAPAIGN, ILLINOIS U pages 275 - 284; FROSETH, B.R. et al.: "Plasmid-mediated reduced phage sensitivity in Streptococcus lactis KR5" * le document en entier * | 1, 3 | |
| X | JOURNAL OF DAIRY SCIENCE. vol. 70, 1987, CHAPAIGN, ILLINOIS U pages 2211 - 2219; LAIBLE, N.J. et al.: "Identification and cloning of plasmid desoxyribonucleic acid coding for abortive phage infection from Streptococcus lactis ssp. diacety lactis KR2 " * le document en entier * | 1-4, 9-11 | |
| X | PLASMID vol. 22, no. 1, 1989, pages 32 - 43; STEELE, J.L & McKAY, L.L: "Conjugal transfer of genetic material by Lactococcus lactis subsp. lactis 11007" * le document en entier * | 1-4, 9-11 | DOMAINES TECHNIQUES RECHERCHES (Int. Cl.5 ) |
| X | PLASMID vol. 23, no. 1, janvier 1990, pages 71 - 75; JOSEPHSEN & KLAENHAMMER: "Stacking of three different restriction and modification systems in Lactococcus lactis by cotransformation" * le document en entier * | 1-4, 9-11 | |
| X | APPLIED AND ENVIRONMENTAL MICROBIOLOGY vol. 53, no. 5, mai 1987, pages 923 - 927; GAUTIER, M. & CHOPIN, M.C: "Plasmid-determined systems for restriction and modification activity and sbortive infection in Streptococcus cremoris" * le document en entier * | 1-4, 9-11 | |

Le présent rapport a été établi pour toutes les revendications

| Lieu de la recherche | Date d'achèvement de la recherche | Examinateur |
|---|---|---|
| LA HAYE | 10 JUILLET 1991 | CHAMBONNET F.J. |

EPO FORM 1503 03.82 (P0402)

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

Office européen
des brevets

# RAPPORT DE RECHERCHE EUROPEENNE

Numero de la demande

EP    91 40 0982
Page 4

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X | APPLIED AND ENVIRONMENTAL MICROBIOLOGY mars 1988, pages 777 - 783; JARVIS, A.W.: "Conjugal transfer in Lactic Streptococci of plasmid-encoded insensitivity to prolate- and small isometric-headed bacteriophages" * le document en entier * | 1-4, 9, 10 | |
| X | REVISTA DE MICROBIOLOGIA vol. 20, no. 2, juin 1989, SAO PAULO, BR pages 197 - 209; SANTOS LERAYER & CHOPIN: "Perfil de plasmidios e resistência a bacteriophagos em estreptococcos lácticos mesophilos" * le document en entier * | 1-4, 9, 10 | |
| X | US-A-4883756 (KLAENHAMMER, T.R. & SANOZKY-DAWES, R. B.) * le document en entier * | 1-4, 9-11 | |

DOMAINES TECHNIQUES RECHERCHES (Int. Cl.5)

Le présent rapport a été établi pour toutes les revendications

| Lieu de la recherche | Date d'achèvement de la recherche | Examinateur |
|---|---|---|
| LA HAYE | 10 JUILLET 1991 | CHAMBONNET F.J. |